(19) Europäisches Patentamt
European Patent Office
Office européen des brevets

(11) Numéro de publication : **0 110 755**
**B1**

(12) **FASCICULE DE BREVET EUROPÉEN**

(45) Date de publication du fascicule du brevet : **01.10.86**

(21) Numéro de dépôt : **83402142.0**

(22) Date de dépôt : **04.11.83**

(51) Int. Cl.[4] : **C 07 D401/04**, C 07 D401/14, C 07 D413/14, A 61 K 31/44// C07D213/57

(54) **Dérivés de la pipéridinedione protecteurs du myocarde présentant une activité antiarythmique, leur procédé de préparation et les médicaments qui contiennent lesdits dérivés.**

(30) Priorité : **08.11.82 FR 8218706**

(43) Date de publication de la demande : **13.06.84 Bulletin 84/24**

(45) Mention de la délivrance du brevet : **01.10.86 Bulletin 86/40**

(84) Etats contractants désignés : **AT BE CH DE FR GB IT LI LU NL SE**

(56) Documents cités :
**EP-A- 0 027 412**
**FR-A- 2 212 147**
**CHEMISCHE BERICHTE, vol. 114, no. 1, 9 janvier 1981, pages 32-48, Verlag Chemie, GmbH, Weinheim, DE H. STAMM et al.: "Einstufensynthese von Pyrrolidonen durch Amidoethylierungen einfacher Ester mit N-Acylaziriden"**

(73) Titulaire : **SANOFI, société anonyme**
**40, Avenue George V**
**F-75008 Paris (FR)**

(72) Inventeur : **Bernhart, Claude**
**144 rue des Muriers**
**F-34980 Saint-Gely du Fesc (FR)**
Inventeur : **Cautreels, Werner**
**5 Impasse des Décurions**
**F-34170 Castelnau-le-Lez (FR)**
Inventeur : **Gautier, Patrick**
**Manavielle**
**F-34660 Cournonterral (FR)**

(74) Mandataire : **Gillard, Marie-Louise et al**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne en tant que produits industriels nouveaux des dérivés de la pipéridinedione-2,6, ainsi que leurs méthodes de préparation et leur application en thérapeutique.

Lesdits dérivés sont substitués en position 3 par un groupe pyridyle et peuvent notamment être utilisés comme protecteurs du myocarde.

Des dérivés de la pipéridinedione-2,6, substitués en position 3 par un groupe phényle ont été décrits dans le brevet FR 2 212 147 et présentés comme possédant une activité antidépressive.

Les nouveaux composés selon l'invention répondent à la formule générale I :

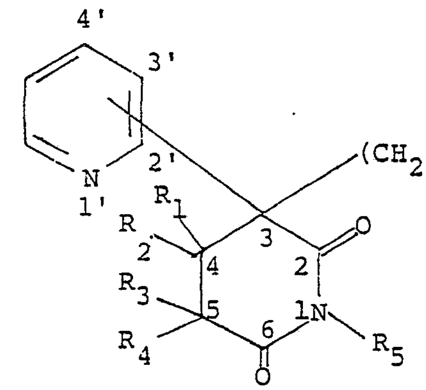

(I)

dans laquelle

R représente un groupe alkyle droit ou ramifié ayant 2 à 5 atomes de carbone, ou encore

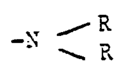

le groupe

représente un groupe morpholino ou pipéridino éventuellement substitué par 1 à 4 groupes méthyle,

n = 2 ou 3,

$R_1$, $R_2$, $R_3$ et $R_4$ considérés indépendamment représentent un atome d'hydrogène ou un groupe alkyle droit ou ramifié ayant 1 à 4 atomes de carbone, ou

$R_3$ et $R_4$ pris ensemble représentent un groupe $(CH_2)_m$ où m = 4 ou 5, ou encore

$R_2$ et $R_3$ pris ensemble représentent un groupe $(CH_2)_p$ où p = 3 ou 4 et, dans ce cas, $R_1$ et $R_4$ sont l'hydrogène,

$R_5$ désigne l'hydrogène ou un groupe alkyle droit ou ramifié ayant 1 à 4 atomes de carbone, enfin, la pipéridinedione substitue le groupe pyridyle en 2', 3' ou 4'.

Les composés (I) fournissent avec les acides organiques ou minéraux des sels solubles. Ces sels avec des acides pharmaceutiquement acceptables font partie intégrante de l'invention.

Les composés (I) possèdent toujours un atome de carbone asymétrique, à savoir l'atome 3 du cycle de la pipéridinedione. Si les substituants $R_1$ et $R_2$, d'une part, ou $R_3$ et $R_4$, d'autre part, sont différents l'un de l'autre, il existe dans la molécule un ou deux autres atomes de carbone asymétriques. Par suite, les composés (I) peuvent exister sous la forme de diastéréo-isomères et d'isomères optiques. Tous ces isomères ainsi que leurs mélanges font partie intégrante de l'invention.

Les composés selon l'invention sont obtenus à partir d'un pyridylacétonitrile selon l'une des méthodes de préparation indiquées ci-après :

Méthode A

Cette méthode est représentée par le schéma réactionnel ci-dessous :

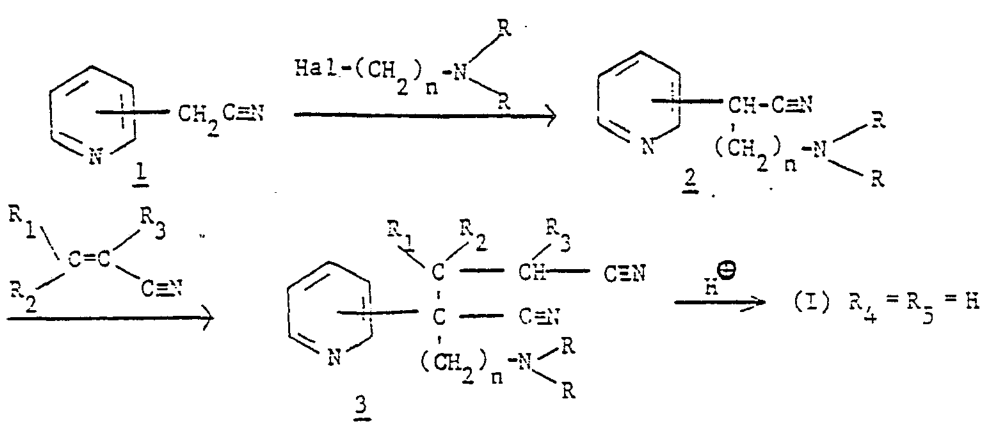

Le pyridylacétonitrile *1* est soumis tout d'abord à une réaction d'alkylation par un composé :

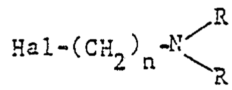

(Hal représentant un halogène) en présence d'une base organique ou minérale pour conduire au composé *2*.

Celui-ci est à nouveau substitué par action d'un nitrile α insaturé :

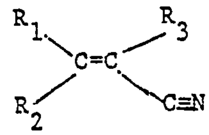

pour conduire au composé *3*. On opère le plus souvent au sein d'un solvant inerte, tel que le tétrahydrofuranne, en présence d'un hydroxyde d'ammonium quaternaire, tel que l'hydroxyde de benzyltriméthylammonium, à une température comprise entre 0 et 30 °C.

On peut également effectuer la réaction en présence d'amidure de sodium au sein de l'amoniac liquide à une température comprise entre — 30 et — 40 °C. Le composé *3* est cyclisé en composé (I), $R_5$ = H, par chauffage en milieu acide. On utilise soit l'acide chlorhydrique en milieu acide acétique, soit l'acide sulfurique concentré ou encore l'acide polyphosphorique à une température comprise entre 100 et 150 °C.

Méthode B

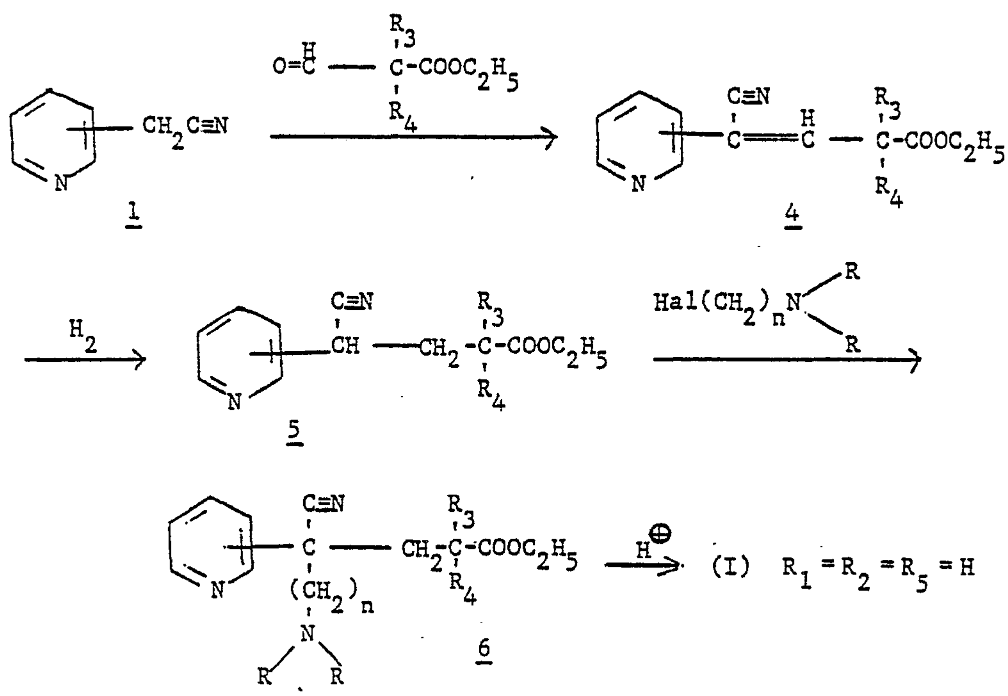


Sur le pyridylacétonitrile *1*, on fait réagir un dérivé carbonylé

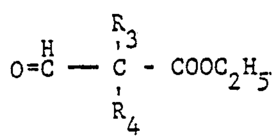

qui conduit au nitrile ester insaturé *4*.

On opère dans un solvant inerte, tel que le benzène, le plus souvent à température d'ébullition du solvant en ajoutant comme catalyseur soit l'acide paratoluènesulfonique, soit la pipéridine et l'acide acétique. Le dérivé *4* est transformé en dérivé saturé correspondant par réduction catalytique. On opère en solution dans l'éthanol en présence d'un catalyseur d'hydrogénation tel que le palladium sur charbon à température ambiante sous pression de l'atmosphère.

Le composé *5* est substitué sur le carbone en α du nitrile par action d'un composé :

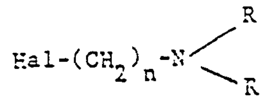

en présence d'un agent de sodation, tel que l'hydrure de sodium, au sein d'un solvant tel que le diméthylformamide. Le plus souvent, on opère à une température comprise entre 20 et 50 °C.

Enfin, le composé *5* est cyclisé en produit (I) $R_5 = H$ par chauffage à une température comprise entre 80 et 120 °C avec de l'acide sulfurique concentré.

Méthode C

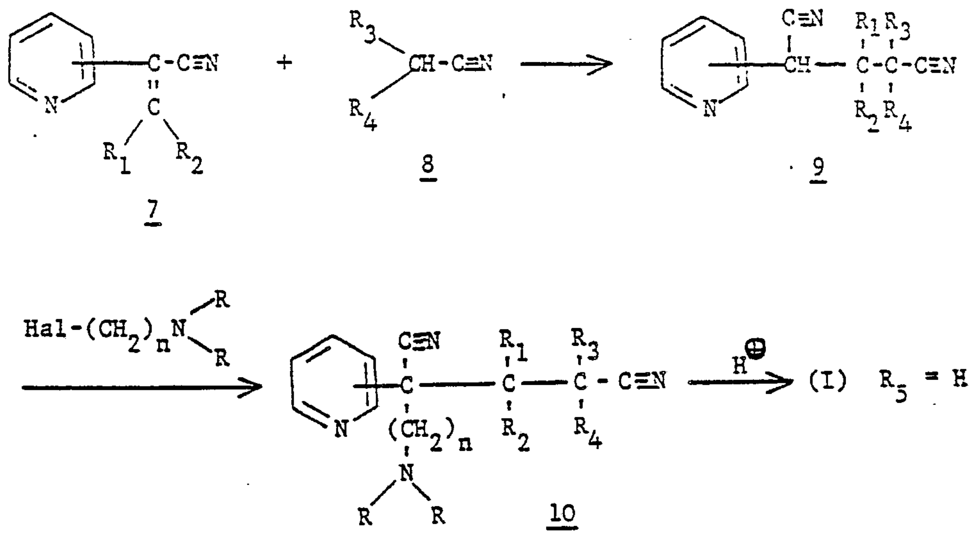


7 8 9

10

La condensation du nitrile *8* sur le nitrile insaturé *7* conduit au dinitrile *9*. La condensation s'effectue en présence de diisopropylamidure de lithium (préparé in situ par action de diisopropylamine sur une solution de butyllithium) à basse température et en solution dans un solvant tel que le tétrahydrofuranne.

On substitue ensuite le dinitrile *9* par action d'un dérivé halogéné

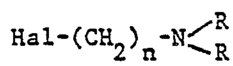

pour obtenir le dinitrile *10*. On opère en présence d'un agent de sodation tel que l'hydrure de sodium au sein d'un solvant inerte tel que le diméthylformamide.

Enfin, on cyclise le dinitrile *10* par action d'un acide minéral concentré tel que l'acide sulfurique, à une température comprise entre 80 et 120 °C.

Méthode D

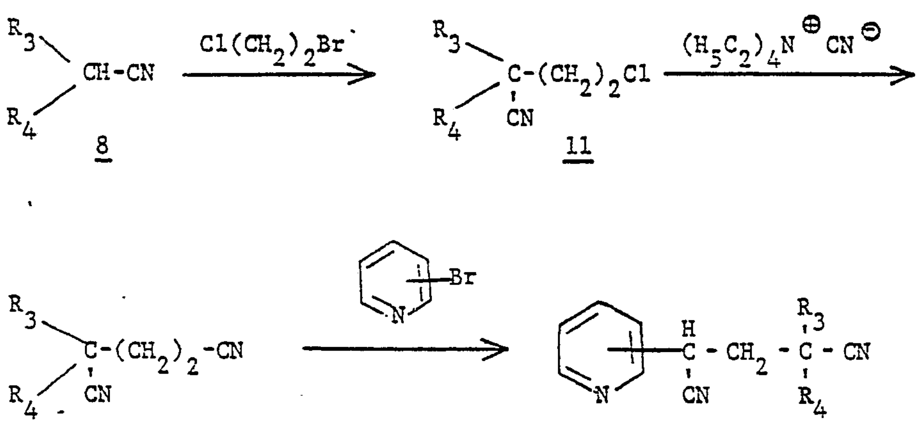


8 11

12 13

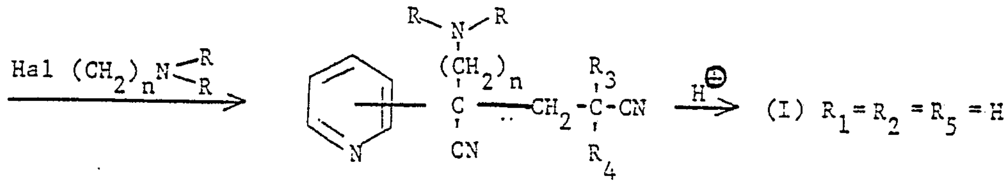


14

On substitue le nitrile *8* par le chloro-1 bromo-2 éthane pour obtenir le nitrile chloré *11.* La substitution s'effectue de préférence en présence de diéthylamidure de lithium (préparé *in situ* par action de la diéthylamine sur le butyllithium) à température ambiante, dans un solvant inerte comme l'éther ou le tétrahydrofuranne.

Par action sur *11* d'un cyanure et de préférence le cyanure de tétraéthylammonium, on obtient le dinitrile *12.* On opère au sein d'un solvant des deux réactifs comme l'acétonitrile à une température de 40 à 60 °C.

On substitue le dinitrile *12* par la bromopyridine pour obtenir le dinitrile *13.* On opère en présence de diisopropylamidure de lithium au sein d'un solvant comme le tétrahydrofuranne et à une température de — 10 à — 20 °C. On substitue une seconde fois le dinitrile *13* par un dérivé halogéné

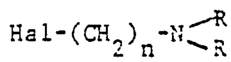

en opérant de façon identique à celle utilisée pour le dinitrile 10 dans la méthode C.

Enfin, par hydrolyse acide comme indiqué pour le dinitrile *10* dans la méthode C, on obtient le composé (I) $R_1 = R_2 = R_5 = H$.

Les composés (I) où $R_5$ est alkyle s'obtiennent à partir des composés où $R_5$ est l'hydrogène et obtenus par l'une des méthodes A, B, C ou D, par alkylation à l'azote selon une méthode connue, par exemple par action d'un halogénure d'alkyle sur le dérivé sodé obtenu par action de l'hydrure de sodium sur le composé (I) $R_5 = H$ au sein d'un solvant comme le diméthylformamide.

Les sels des composés (I) s'obtiennent par les méthodes habituelles de salification.

Enfin, lorsque $R_1$ et $R_2$, d'une part, ou $R_3$ et $R_4$, d'autre part, sont différents, les produits (I) existent sous la forme de diastéréo-isomères. Les méthodes A, B, C ou D conduisent à un mélange de ces diastéréo-isomères qui peuvent être séparés par les méthodes habituelles et, en particulier, par chromatographie.

Sur chacun des diastéréo-isomères, une étude de résonance magnétique nucléaire a permis de déterminer la configuration spatiale de ces stéréo-isomères.

Les exemples suivants nullement limitatifs sont donnés à titre d'illustration pour la préparation des composés selon l'invention.

Dans les exemples qui suivent pour désigner les diastéréo-isomères, on utilisera la nomenclature de l'IUPAC — section E (recommandations de 1974) et, en particulier la règle E-5.3.

Exemple 1

Méthyl-5(e) (diisopropylamino-2 éthyl)-3(e) (pyridyl-2)-3(a) pipéridinedione-2,6, dichlorhydrate (SR 40976)

R = 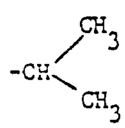 ; $n = 2$ ; $R_1 = R_2 = R_4 = H$ ; (I)

$R_3 = —CH_3$ équatorial ; $R_5 = H$

a) Diisopropylamino-4 (pyridyl-2)-2 butane nitrile

On mélange 80 g de pyridyl-2 acétonitrile, 8,8 g de chloro-1 diisopropylamino-2 éthane et 2,7 g de chlorure de benzyltriéthylammonium. En maintenant la température au-dessous de 35 °C, on ajoute peu à peu 350 ml d'une solution aqueuse de soude à 50 %.

On chauffe le mélange à 35 °C pendant 5 h. Après refroidissement, on ajoute de l'eau et extrait avec de l'éther. On sépare la phase organique, sèche sur sulfate de sodium puis évapore le solvant à siccité.

Par distillation du résidu, on obtient un liquide jaune (94 g) ; Eb/0,6 mmHg : 132-134 °C.

b) Diisopropylamino-2 éthyl)-2 méthyl-4 (pyridyl-2)-2 pentane dinitrile

A une solution de 17,3 g du nitrile obtenu ci-dessus dans 70 ml de tétrahydrofuranne, on ajoute 3,2 ml

d'une solution à 40 % d'hydroxyde de benzyltriméthylammonium dans le méthanol. On ajoute goutte à goutte la solution de 5,2 g de méthacrylonitrile dans 35 ml de tétrahydrofuranne et laisse ensuite sous agitation durant 1 h.

On évapore le solvant à siccité et reprend le résidu dans l'eau et l'éther. On sépare la phase éthérée et réextrait la phase aqueuse avec de l'éther. On réunit les extraits éthérés, lave avec de l'eau et sèche sur sulfate de sodium. On évapore le solvant à siccité.

On obtient un liquide jaune orangé (22,7 g) utilisé tel quel pour la suite de l'opération.

c) SR 40976

On chauffe au reflux pendant 2 h le mélange de 22,7 g du dinitrile obtenu précédemment, 136 ml d'acide chlorhydrique (d = 1,19) et 136 ml d'acide acétique.

On évapore à siccité sous vide et reprend le résidu dans un peu d'eau. On ajoute une solution saturée de bicarbonate de sodium et extrait 3 fois avec du chloroforme. On réunit les extraits organiques, sèche sur sulfate de sodium et évapore le solvant à siccité.

On obtient 16 g de produit brut constitué par le mélange des deux diastéréo-isomères. On chromatographie sur une colonne d'alumine. En éluant par le mélange acétate d'éthyle-pentane 30-70 vol/vol, on obtient d'abord l'un des diastéréo-isomères pur (5,35 g).

L'étude du spectre de RMN du produit montre que, dans ce diastéréo-isomère, le méthyl en 5 et le groupe (diisopropylamino-2 éthyl) en 3 sont équatoriaux, alors que le groupe pyridyl-2 est axial.

Dichlorhydrate

On dissout 5,02 g du diastéréo-isomère pur ci-dessus dans 50 ml d'éthanol absolu et ajoute 3,07 g d'acide chlorhydrique d = 1,19 dissous dans 50 ml d'éthanol absolu. On évapore le solvant et reprend le résidu dans l'acétone. Le dichlorhydrate cristallise sous forme de cristaux incolores. On essore et lave avec un peu d'acétone. Poids : 6,26 g ; F. 157-160 °C. Le dichlorhydrate cristallise avec 1 molécule d'eau.

Exemple 2

(Diisopropylamino-2 éthyl)-3 (pyridyl-2)-3 pipéridinedione-2,6 (SR 41299)

R = 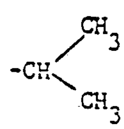 ; n = 2 (I)

$R_1 = R_2 = R_3 = R_4 = R_5 = H$

a) Diisopropylamino-2 (pyridyl-2)-2 pentanedinitrile

On opère comme dans l'exemple 1b), à partir du nitrile de l'exemple 1a), mais en remplaçant le méthacrylonitrile par l'acrylonitrile.

b) SR 41299

On chauffe à 115 °C, pendant 1 h 30, 11,6 g du composé obtenu ci-dessus et 110 g d'acide polyphosphorique. Après refroidissement, on dissout le milieu réactionnel dans l'eau et on alcalinise avec du carbonate de potassium. On extrait avec de l'acétate d'éthyle, sèche sur sulfate de sodium puis évapore le solvant à siccité.

On chromatographie le résidu sur une colonne d'alumine en éluant par un mélange acétate d'éthyle-pentane d'abord 50-50 vol/vol puis 75-25 vol/vol. On obtient ainsi une huile qui cristallise lentement. On recristallise dans l'éther isopropylique et obtient des cristaux incolores (5,25 g), F. 96-97 °C.

Exemple 3

Isopropyl-5(e) (diisopropylamino-2 éthyl)-3(e) (pyridyl-2)-3(a)-pipéridinedione-2,6 (SR 41411)

(I) = 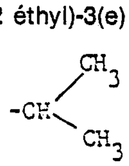 ; n = 2 ; $R_1 = R_2 = R_4 = H$ ;

$R_3$ = 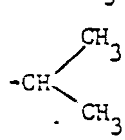 ; équatorial : $R_5 = H$

a) (Diisopropylamino-2 éthyl)-2 isopropyl-4 (pyridyl-2)-2 pentane-dinitrile

On opère comme dans l'exemple 1b), à partir du nitrile de l'exemple 1a), mais en remplaçant le méthacrylonitrile par une quantité équivalente d'isopropyl-2 acrylonitrile

b) SR 41411

On dissout 17 g du dinitrile préparé ci-dessus dans 100 ml d'acide sulfurique concentré (d : 1,83), puis on chauffe à 100-110 °C pendant 1 h. On verse le mélange réactionnel sur de la glace et alcalinise la solution par de la soude en solution à 40 %. On extrait avec de l'acétate d'éthyle et sèche la solution organique sur sulfate de sodium.

On évapore le solvant à siccité et chromatographie le résidu sur une colonne d'alumine. En éluant avec le mélange acétate d'éthyle-pentane 20/80 vol/vol, on obtient tout d'abord 5,3 g de diastéréo-isomère pur puis 3,3 g d'un mélange des deux diastéréo-isomères.

On recristallise le diastéréo-isomère pur dans l'éther isopropylique ; F. 123-125 °C.

En opérant comme dans l'exemple 3, mais en faisant varier l'acrylonitrile utilisé à l'étape a), on obtient :

— avec l'isobutyl-2 acrylonitrile : l'isobutyl-5(e) (disopropylamino-2 éthyl)-3(e) (pyridyl-2)-3(a) pipéridinedione-2,6 (SR 41463), F. 112-114 °C (hexane)

— avec l'éthyl-2 acrylonitrile : l'éthyl-5(e) (diisopropylamino-2 éthyl)-3(e) (pyridyl-2)-3(a) pipéridinedione-2,6 (SR 41575) F. 107-109 °C (hexane).

## Exemple 4

Tertiobutyl-5 (diisopropylamino-2 éthyl)-3 (pyridyl-2)-3 pipéridine-dione-2,6

a) (Diisopropylamino-2 éthyl)-2 tertiobutyl-4 (pyridyl-2)-2 pentane-dinitrile

On opère comme dans l'exemple 1b) en remplaçant le méthacrylonitrile par du tertiobutyl-2 acrylonitrile en quantité équivalentre.

b) Cyclisation

On opère comme dans l'exemple 3b), à partir du produit obtenu ci-dessus.

Le produit brut de réaction est chromatographié sur une colonne d'alumine (25 g d'alumine par gramme du produit) en éluant par le mélange acétate d'éthyle-pentane, 15-85 vol/vol.

On obtient tout d'abord le premier diastéréo-isomère : tertiobutyl-5(e) (diisopropylamino-2 éthyl)-3(e) (pyridyl-2)-3(a) pipéridinedione-2,6 (SR 41494) ; poids : 8,7 g, F. 101-102 °C (hexane). Puis, après un mélange des deux isomères (2,7 g), on isole le second diastéréo-isomère pur : tertiobutyl-5(e) (diisopropylamino-2 éthyl)-3(a) (pyridyl-2)-3(e) pipéridinedione-2,6 (SR 41584) ; poids : 5,1 g, f. 102-103 °C (hexane).

## Exemple 5

Diméthyl-4,4 (diisopropylamino-2 éthyl)-3 (pyridyl-2)-3 pipéridinedione-2,6 (SR 41694)

R = 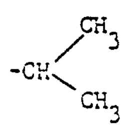 ; n = 2 ; $R_1 = R_2 = CH_3$ ; (I)

$R_3 = R_4 = R_5 = H$

a) (Diisopropylamino-2 éthyl)-2 diméthyl-3,3 (pyridyl-2)-2 pentanedinitrile

On refroidit à — 40 °C le mélange de 4,7 g d'amidure de sodium et 400 ml d'ammoniac liquide, puis on ajoute la solution de 24,5 g de diisopropylamino-4 (pyridyl-2)-2 butane nitrile (exemple 1a), dans 30 ml d'éther anhydre en maintenant la température entre — 40 et — 33 °C. On agite 15 min à cette température puis on ajoute la solution de 9 g de diméthyl-3,3 acrylonitrile dans 40 ml d'éther, toujours à la même température. On laisse remonter lentement la température jusqu'à la température ambiante (durée 5 h environ). On ajoute 200 ml d'éther puis on additionne de l'eau goutte à goutte. On sépare la phase éthérée et réextrait la phase aqueuse avec de l'éther. On réunit les extraits éthérés et sèche sur sulfate de sodium. On évapore le solvant à siccité et chromatographie le résidu sur colonne d'alumine avec le mélange éluant acétate d'éthyle-pentane 2,5-100 vol/vol.

On élimine en tête les produits n'ayant pas réagi, puis on obtient 12,5 g du produit attendu utilisé tel quel pour la cyclisation.

b) SR 41694

On effectue la cyclisation par l'acide sulfurique comme indiqué dans l'exemple 3b). On obtient le produit attendu sous forme de cristaux incolores, F. 105-106 °C (cyclohexane-hexane).

Exemple 6

(Diisopropylamino-2 éthyl)-4 (pyridyl-2)-4 décahydroisoquinoléine-dione-1,3 (SR 42420)

R = 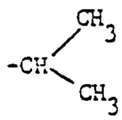 ; $n = 2$ ; $R_1 = R_4 = H$ ; $R_2 + R_3 = (CH_2)_4$ (I)

a) Cyano-2 cyclohexyl)-2 diisopropylamino-4 (pyridyl-2)-2 butyronitrile.

A la solution de 36,75 g de diisopropylamino-4 (pyridyl-2)-2 butyronitrile (exemple 1a) dans 300 ml de tétrahydrofuranne, on ajoute à température ambiante 71,7 g de Triton B, puis 15,9 g de cyclohexène-1 carbonitrile dissous dans 100 ml de tétrahydrofuranne. On laisse une nuit sous agitation à température ambiante (20 °C environ), puis on évapore le solvant à siccité. On reprend le résidu dans l'eau et extrait trois fois avec de l'éther. On évapore le solvant et chromatographie le résidu sur une colonne d'alumine. En éluant avec un mélange pentane-acétate d'éthyle 80-20 vol/vol, on obtient 20 g du produit attendu utilisé tel quel pour la cyclisation.

b) SR 42420

On chauffe à 100 °C pendant 1 h le mélange de 20 g du produit obtenu ci-dessus et 200 ml d'acide sulfurique concentré (d = 1,83). Après refroidissement, on verse sur de la glace, puis on alcalinise avec une solution de soude à 40 % en refroidissant de façon que la température du mélange ne dépasse pas 30 °C. On extrait trois fois avec de l'acétate d'éthyle et sèche les extraits organiques sur sulfate de sodium. On évapore le solvant à siccité et chromatographie le résidu sur colonne d'alumine. En éluant avec le mélange pentane-acétate d'éthyle 90-10 vol/vol, on obtient le produit attendu (4,3 g) ; F. 159-160 °C (éther isopropylique).

Exemple 7

(Diisopropylamino-2 éthyl)-3 diméthyl-5,5 (pyridyl-2)-3 pipéridinedione-2,6 dichlorhydrate (SR 41298)

R = 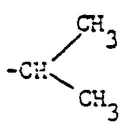 ; $n = 2$ ; $R_1 = R_2 = H$ ; (I)

$R_3 = R_4 = CH_3$ ; $R_5 = H$

a) Cyano-4 diméthyl-2,2 (pyridyl-2)-4 butène-3 oate d'éthyle

Dans un ballon équipé d'un séparateur d'eau, on introduit 12,8 g de pyridyl-2 acétonitrile, 22 g de formyl-2 méthyl-2 propionate d'éthyle, 500 ml de benzène, 1 ml d'acide acétique et 0,2 ml de pipéridine. On porte au reflux jusqu'à ce qu'il ne se sépare plus d'eau (environ 69 h) puis, après refroidissement, on lave la solution organique avec une solution de bicarbonate de sodium. On sèche la solution sur sulfate de sodium, puis évapore le solvant à siccité.

On utilise le produit tel quel pour l'étape suivante.

b) Cyano-4 diméthyl-2,2 (pyridyl-2)-4 butyrate d'éthyle

On dissout le produit obtenu ci-dessus dans 600 ml d'éthanol et ajoute 15 g de palladium sur charbon à 5 %. On hydrogène à température ambiante sous 1 atmosphère. On filtre le catalyseur et évapore le solvant à siccité.

On distille le résidu sous vide poussé.

E./0,001 mmHg ; 117-120 °C ; poids : 20,8 g

c) Cyano-4 diméthyl-2,2 (diisopropylamino-2 éthyl)-4 (pyridyl-2)-4 butyrate d'éthyle

On place dans un ballon sous atmosphère d'azote 1,4 g d'hydrure de sodium et 20 ml de

diméthylformamide. On ajoute goutte à goutte la solution de 9,8 g du composé obtenu en b), dans 20 ml de diméthylformamide. On laisse 1 h sous agitation à température ambiante puis on ajoute la solution de 7,2 g de chloro-1 diisopropylamino-2 éthane dans 20 ml de diméthylformamide et on laisse 2 h sous agitation à température ambiante.

On évapore le solvant à siccité et reprend le résidu dans l'éther. On lave la solution avec de l'eau, sèche sur sulfate de sodium et évapore le solvant à siccité.

Le produit ainsi obtenu est utilisé tel quel dans l'étape suivante.

d) SR 41298

Le composé obtenu ci-dessus est dissous dans 130 ml d'acide sulfurique concentré (d = 1,83) et le mélange réactionnel est chauffé pendant 1 h à 100-110 °C. On verse le mélange sur de la glace et alcalinise la solution par addition d'une solution aqueuse de carbonate de potassium. On extrait avec de l'acétate d'éthyle et sèche la solution sur sulfate de sodium.

On évapore le solvant à siccité et chromatographie le résidu sur une colonne d'alumine. En éluant d'abord avec le mélange acétate d'éthyle-pentane 50-50 vol/vol, puis avec de l'acétate d'éthyle pur, on obtient 11,7 g du produit attendu.

Dichlorhydrate

On dissout 10 g de la base ci-dessus dans 100 ml d'éthanol et ajoute 5,8 g d'acide chlorhydrique concentré (d = 1,18). On évapore à siccité et ajoute de l'éther. Le résidu cristallise.

On recristallise dans l'alcool absolu. On obtient des cristaux incolores (7,3 g) ; F. 203-204 °C. Le chlorhydrate cristallise avec 1 molécule d'eau.

Exemples 8 à 21

En opérant comme dans l'exemple 7, mais en faisant varier le dérivé carbonylé utilisé au paragraphe a) et/ou le dérivé halogéné utilisé au paragraphe c), on obtient de la même façon les composés (I) réunis dans le tableau 1 suivant.

(Voir tableau 1 pp. 10 et 11)

Tableau 1

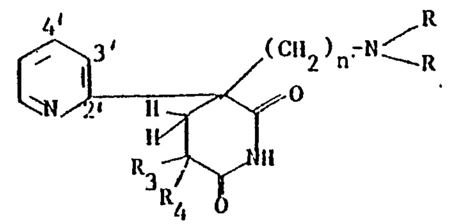

| Exemple n° | N° de code SR | Position substitution pyridine | $-N{<}^{R}_{R}$ | n | $R_3$ | $R_4$ | Base ou sel | Point de fusion, °C (solvant de cristallisation) |
|---|---|---|---|---|---|---|---|---|
| 8 | 41338 | 2' | $-N[CH(CH_3)_2]_2$ | 2 | $(CH_2)_4$ | | Base | 86-87 (éther isopropylique) |
| 9 | 41612 | 2' | $-N[CH_2CH_2CH_3]_2$ | 2 | $-CH_3$ | $-CH_3$ | Base | 73-74 (hexane) |
| 10 | 41620 | 2' | 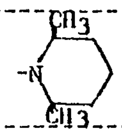 | 2 | $-CH_3$ | $-CH_3$ | Base | 153-154 (acétate d'éthyle |
| 11 | 41639 | 2' | 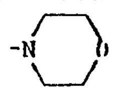 | 2 | $-CH_3$ | $-CH_3$ | Base | 163-164 (acétate d'éthyle) |
| 12 | 41692 | 2' | $-N[CH(CH_3)_2]_2$ | 2 | $-CH_2CH_3$ | $-CH_2CH_3$ | Dichlorhydrate avec 1,5 $H_2O$ | 124-126 (isopropanol) |
| 13 | 41700 | 2' | " | 3 | $-CH_3$ | $-CH_3$ | Base | 94-95 (éther isopropylique) |
| 14 | 41720 | 2' | 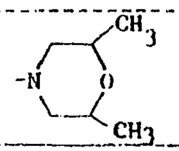 | 2 | $-CH_3$ | $-CH_3$ | Base | 119-120 (éther isopropylique) |

Tableau 1 (suite)

| Exemple n° | n° de code SR | Position substitution pyridine | 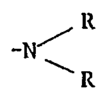 | n | $R_3$ | $R_4$ | Base ou sel | Point de fusion, °C (solvant de cristallisation) |
|---|---|---|---|---|---|---|---|---|
| 15 | 41770 | 2' | $-N-(CH_2CH_3)_2$ | 2 | $-CH_3$ | $-CH_3$ | Base | 83-85 (hexane) |
| 16 | 41811 | 2' | $-N[CH(CH_3)(C_2H_5)]_2$ | 2 | $-CH_3$ | $-CH_3$ | Base | 65-67 (hexane) |
| 17 | 41821 | 2' | 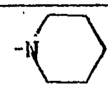 | 2 | $-CH_3$ | $-CH_3$ | Base | 136-137 (éther isopropylique) |
| 18 | 42138 | 4' | $-N[CH(CH_3)_2]_2$ | 2 | $-CH_3$ | $-CH_3$ | Base | 132-134 (éther isopropylique) |
| 19 | 42150 | 3' | " | 2 | $-CH_3$ | $-CH_3$ | Base | 51-53 (hexane) |
| 20 | 42151 | 2' | 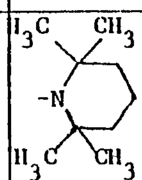 | 2 | $-CH_3$ | $-CH_3$ | Base | 198-199 (acétate d'éthyle) |
| 21 | 42241 | 2' | 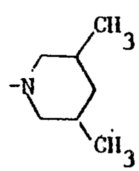 | 2 | $-CH_3$ | $-CH_3$ | Base | 145-146 (éther isopropylique) |

Exemple 22

(Diisopropylamino-2 éthyl)-3 (pyridyl-2)-3 tétraméthyl-4,4,5,5 pipéridinedione-2,6 (SR 42436)

R = 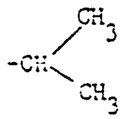 ; $n = 2$ ; $R_1 = R_2 = R_3 = R_4 = CH_3$ ; $R_5 = H$ (I)

a) (Pyridyl-2)-4 tétraméthyl-2,2,3,3 pentane dinitrile-1,5

A 68 ml d'une solution de n-butyllithium 1,6 M dans l'hexane refroidie à — 20 °C, on ajoute en agitant, sous atmosphère d'azote, la solution de 11,1 g de diisopropylamine dans 100 ml de tétrahydrofuranne. On refroidit ensuite à — 70 °C et on ajoute la solution de 6,9 g d'isobutyronitrile dans 100 ml de tétrahydrofuranne. Après 15 min d'agitation à — 70 °C, on ajoute la solution de 17,4 g de méthyl-3 (pyridyl-2)-2 butène-2 nitrile dans 100 ml de tétrahydrofuranne.

Après la fin de l'addition, on laisse remonter lentement la température jusqu'à la température ambiante, puis on évapore le solvant à siccité. On reprend le résidu dans l'eau et extrait trois fois avec de l'éther. On sèche les extraits organiques sur sulfate de sodium et évapore le solvant. On distille le résidu sous pression réduite et on recueille 11,3 g d'un liquide rouge.

E./1,5mm Hg : 160-170 °C.

b) Diisopropylamino-6 (pyridyl-2)-4 cyano-4 tétraméthyl-2,2,3,3 hexane nitrile

Sous atmosphère d'azote, on ajoute 2,4 g d'une suspension à 55 % d'hydrure de sodium dans l'huile dans 50 ml de diméthylformamide. On ajoute ensuite goutte à goutte la solution de 11,3 g du dinitrile obtenu précédemment dans 50 ml de diméthylformamide. On agite pendant 30 min à température ambiante, puis on ajoute la solution de 9 g de chloro-1 diisopropylamino-2 éthane dans 50 ml de diméthylformamide. On agite pendant 4 h à température ambiante, puis on évapore le solvant sous vide. On reprend le résidu dans l'eau et extrait avec de l'acétate d'éthyle. On sèche la solution sur sulfate de sodium et évapore le solvant sous vide. On chromatographie le résidu sur colonne d'alumine. En éluant avec le mélange pentane-acétate d'éthyle 90-10 vol/vol, on obtient un liquide visqueux (16 g) utilisé tel quel pour la cyclisation.

c) SR 42436

On chauffe à 100 °C pendant 1 h 16 g du composé obtenu précédemment et 150 ml d'acide sulfurique concentré (d = 1,83). On ajoute 300 ml d'eau et porte au reflux pendant 12 h. Après refroidissement, on alcalinise par addition de solution de soude à 40 % en refroidissant extérieurement de façon à maintenir la température du mélange réactionnel en dessous de 30 °C.

On extrait trois fois avec de l'acétate d'éthyle et on sèche les extraits organiques sur sulfate de sodium. On évapore le solvant et chromatographie sur colonne d'alumine. En éluant avec un mélange pentane-acétate d'éthyle 80-20 vol/vol, on obtient un liquide épais qui cristallise en présence d'éther isopropylique.

Après recristallisation dans l'éther isopropylique, on obtient des cristaux incolores (4,1 g) ; F. 89-90 °C.

En opérant de la même façon, mais en remplaçant, dans l'étape a), le méthyl-3 (pyridyl-2)-2 butène-2 nitrile par une quantité équivalente de (pyridyl-2)-2 butène-2 nitrile, on obtient de la même façon le (diisopropylamino-2 éthyl)-3 (pyridyl-2)-3 triméthyl-4,5,5-pipéridinedione-2,6 ; cristaux incolores ; F. 93-94 °C (éther isopropylique) (SR 42480).

Exemple 23

(Diisopropylamino-2 éthyl)-3 (pyridyl-2)-3 dipropyl-5,5 pipéridinedione-2,6 (SR 42481)

R = 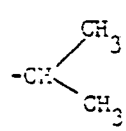 ; = 2 ; $R_1 = R_2 = R_5 = H$ ; $R_3 = R_4 = —CH_2—CH_2—CH_3$

a) (Chloro-2 éthyl)-2 propyl-2 pentane-nitrile

A la solution (245 ml) de n-butyllithium 1,6 M dans l'hexane, on ajoute goutte à goutte sous atmosphère d'azote et en agitant la solution de 28,8 g de diéthylamine dans 100 ml d'éther anhydre. Après 1 h, on ajoute à température ambiante la solution de 49,2 g de propyl-2 pentane nitrile dans 100 ml d'éther

anhydre, puis, après retour à température ambiante, on ajoute la solution de 49,1 g de bromo-1 chloro-2 éthane dans 700 ml d'éther anhydre. On porte au reflux pendant 15 h puis, en refroidissant le mélange réactionnel dans un bain de glace, on ajoute de l'eau. On sépare la phase organique, lave avec de l'eau, puis avec un solution diluée d'acide chlorhydrique et à nouveau avec de l'eau. On sèche sur sulfate de sodium et évapore le solvant. On distille le résidu et on obtient un liquide E./20 mm Hg : 140-150 °C.

b) Cyano-4 propyl-4 heptane-nitrile.

On chauffe à 50 °C pendant 16 h le mélange de 5,62 g du nitrile obtenu en a), 4,68 g de cyanure de tétraéthylammonium dans 15 ml d'acétonitrile.

On évapore le solvant à siccité et reprned le résidu dans l'éther. On filtre la solution, évapore le solvant et distille le résidu. On obtient un liquide jaune (2,9 g). E./20 mm Hg : 160-170 °C.

c) Cyano-4 propyl-4 (pyridyl-2)-2 heptane nitrile.

A 38 ml d'une solution 1,6 M de n-butyllithium dans l'hexane refroidie à — 10 °C, on ajoute sous atmosphère d'azote la solution de 6,3 g de diisopropylamine dans 100 ml de tétrahydrofuranne sec. On ajoute ensuite à une température comprise entre — 20 et — 10 °C la solution de 10 g du composé obtenu en b) dans 100 ml de tétrahydrofuranne anhydre. Après 15 min d'agitation, on ajoute à — 20 °C la solution de 9,1 g de bromo-2 pyridine dans 100 ml de tétrahydrofuranne anhydre. On laisse ensuite la température du mélange remonter lentement jusqu'à température ambiante, puis on évapore le solvant. On reprend le résidu par de l'eau et extrait trois fois avec de l'éther. On sèche la solution organique sur sulfate de sodium et évapore le solvant. On chromatographie le résidu sur colonne d'alumine. En éluant avec le mélange hexane-acétate d'éthyle 90-10 vol/vol, on obtient un liquide épais (7 g).

d) Cyano-4 (diisopropylamino-2 éthyl)-2 propyl-4 (pyridyl-2)-2 heptane nitrile.

A la solution de 4 g du produit obtenu ci-dessus dans 20 ml de diméthylformamide, on ajoute sous atmosphère d'azote 0,8 g d'une suspension à 55 % d'amidure de sodium dans l'huile. On agite 30 min à température ambiante, puis on ajoute 2,95 g de chloro-1 diisopropylamino-2 éthane dissous dans 20 ml de diméthylformamide.

On agite 2 h à température ambiante, puis on évapore le solvant sous vide et reprend le résidu dans l'eau. On extrait avec de l'éther et sèche la solution sur sulfate de sodium. On évapore le solvant à siccité et utilise le résidu directement pour l'étape suivante (6,6 g).

e) SR 42481

On porte à 100 °C pendant 1 h 6,6 g du composé obtenu en d) et 100 ml d'acide sulfurique concentré (d = 1,83). On ajoute ensuite 200 ml d'eau et porte 1 h au reflux.

On alcalinise avec une solution de soude à 40 % en maintenant la température du mélange au-dessous de 30 °C.

On extrait avec de l'éther, sèche la solution sur sulfate de sodium, puis évapore le solvant. On chromatographie le résidu sur colonne d'alumine. En éluant avec le mélange hexane-acétate d'éthyle 90-10 vol/vol, on obtient 5 g d'un liquide visqueux.

Spectre de RMN enregistré à 250 MHz en solution dans le deutérochloroforme :

1H à 8,55 ppm : (doublet de doublet, $J_1$ = 5 Hz, $J_2$ = 2 Hz, $H_6$ pyridine),
1H à 8,08 ppm : (singulet élargi —N$\underline{H}$—),
1H à 7,62 ppm : (triplet de doublet, $J_1$ = 7 Hz, $J_2$ = 2 Hz, $H_4$ pyridine),
1H à 7,18 ppm : (multiplet, $H_5$ pyridine),
2H à 2,90 ppm : (septuplet, J = 7 Hz, $2CH(C\underline{H}_3)_2$),

2H à 2,65 ppm : (multiplet, 1 $H_4$ et 1 $\underline{CH_2}N<$),

4H à 2,00 ppm : (multiplet, 1 $H_4$ et 3 $-\underline{CH_2}-\underline{CH_2}-N<$),

2H à 1,5 ppm : (multiplet
3H à 1,25 ppm : (multiplet } 2 chaînes $—CH_2CH_2—CH_3$,
5H à 0,95 ppm : (multiplet
12H à 0,90 ppm : (doublet J = 7 Hz, 2 $CH(C\underline{H_3})_2$
1H à 0,5 ppm : (multiplet, chaîne propyle),
3H à 0,36 ppm : (triplet, J = 7 Hz, $—CH_2CH_2—CH_3$)

Exemple 24

Diméthyl-1,5(e) (diisopropylamino-2 éthyl)-3(e) (pyridyl-2)-3(a) dichlorhydrate (SR 41297)

R = 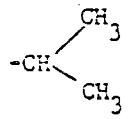 ; n = 2 ; $R_1 = R_2 = R_4 = H$ ; (I)

$R_3 = CH_3$ équatorial ; $R_5 = CH_3$

On met en suspension sous atmosphère d'azote 0,7 g d'hydrure de sodium dans 20 ml de diméthylformamide, puis on ajoute goutte à goutte une solution de 7,2 g du composé de l'exemple 1 dans 20 ml de diméthylformamide.

Après 1 h d'agitation à température ambiante, on ajoute goutte à goutte la solution de 3,0 g d'iodure de méthyle dans 10 ml de diméthylformamide. On agite à nouveau 1 h à température ambiante puis on évapore le solvant à siccité et reprend le résidu dans l'éther. On lave la solution organique avec de l'eau, sèche sur sulfate de sodium et évapore à siccité.

On chromatographie le résidu sur colonne d'alumine en éluant avec le mélange acétate d'éthyle-pentane, 15-85 vol/vol.

On obtient une huile (4,9 g).

Dichlorhydrate

On dissout 4,75 g de la base dans 50 ml d'alcool absolu et ajoute 2,7 g d'acide chlorhydrique (d = 1,18). On évapore à siccité et on ajoute de l'éther. On recristallise dans l'isopropanol. On obtient des cristaux incolores (5 g) ; F. : 167-169 °C. Le dichlorhydrate cristallise avec 0,75 molécule d'eau.

Les produits de l'invention ont été étudiés en pharmacologie, en particulier en vue de mettre en évidence leurs propriétés antiarythmiques.

Protocole

Le pouvoir antiarythmique de ces molécules a été apprécié sur un modèle animal d'arythmie ventriculaire.

Des chiens bâtards sont anesthésiés puis soumis à la mise en place, par cathétérisme rétrograde, d'une spire métallique dans le lit coronarien. Dans le même temps, un micro-émetteur modulateur de fréquence est fixé au dos de l'animal et relié à deux électrodes précordiales.

L'animal remis dans son box accuse alors une thrombose progressive de l'artère interventriculaire antérieure. Ainsi, se constitue un infarctus myocardique localisé et transmural, générateur d'une activité électrique anormale mais répétitive : tachycardie ventriculaire.

Dans cet état, 16 à 24 h après la pose de la spire, les drogues sont administrées *per os* et le système télémétré permet de suivre en temps réel l'évolution de la disrythmie du chien vigile.

Un comptage des complexes systoliques sinusaux et pathologiques est assuré en permanence par des procédés électroniques. Ainsi, peut-on quantifier la qualité et la durée d'action du produit et observer le comportement de l'animal.

Résultats

Un produit est considéré comme actif lorsqu'il supprime 60 % au moins des complexes anormaux ou lorsqu'il rétablit le rythme sinusal.

Les résultats obtenus avec divers produits de l'invention, après administration à la dose de 50 mg/kg *per os*, sont réunis dans le tableau 2 ci-après.

Dans chaque cas, on a indiqué le nombre des expriences et la durée d'activité du produit considéré.

Ces résultats montrent que les produits selon l'invention sont doués d'une forte activité sur la dysryhtmie avec une durée d'action prolongée chez certains d'entre eux.

Par ailleurs, les produits (I) sont peu toxiques et spécialement aucun signe de toxicité n'a pu être mis en évidence aux doses où ils sont actifs sur les dysrythmies.

Par suite, les produits (I) peuvent être utilisés en thérapeutique humaine comme protecteurs du myocarde pour la correction des troubles du rythme ventriculaire d'origine ischémique.

Les produits pourront être présentés sous les formes galéniques correspondant à la voie orale (comprimés, gélules, etc.) et à la voie parentérale (ampoules injectables).

La dose nécessaire à la restauration du rythme sinusal chez l'homme est comprise entre 50 et 150 mg par voie intraveineuse et entre 400 et 800 mg par voie orale, par jour, environ.

Tableau 2

| N° de code du produit | Nombre d'animaux | Durée de l'activité sur la tachycardie ventriculaire |
|---|---|---|
| SR 40976 | 2 | 2 h 45 et 11 h 35 |
| SR 41297 | 1 | 1 h 40 |
| SR 41298 | 3 | supérieure à 24 h les trois fois |
| SR 41299 | 2 | 3 h 20 et 3 h 50 |
| SR 41338 | 2 | 8 h et plus de 24 h |
| SR 41411 | 2 | 3 h 10 et 10 h |
| SR 41463 | 2 | supérieure à 24 h les deux fois |
| SR 41575 | 3 | de 8 h à plus de 24 h |
| SR 41584 | 1 | supérieure à 24 h |
| SR 41612 | 1 | supérieure à 24 h |
| SR 41620 | 2 | supérieure à 24 h les deux fois |
| SR 41639 | 1 | 1 h 40 |
| SR 41694 | 1 | 1 h 50 |
| SR 41700 | 2 | supérieure à 24 h les deux fois |
| SR 41494 | 1 | 9 h |
| SR 41770 | 1 | 5 h |
| SR 41811 | 1 | 7 h |

A titre d'exemple, on indique la préparation galénique suivante :

Comprimés

| | |
|---|---|
| SR 41298 | 0,200 g |
| Cellulose microcristalline | 0,140 g |
| Lactose | 0,140 g |
| Stéarate de magnésium | 0,020 g |
| | 0,500 g |

**Revendications** (pour les Etats contractants : (BE, CH, DE, FR, GB, IT, LI, LU, NL, SE)

1. Dérivés de la pipéridinedione répondant à la formule générale I :

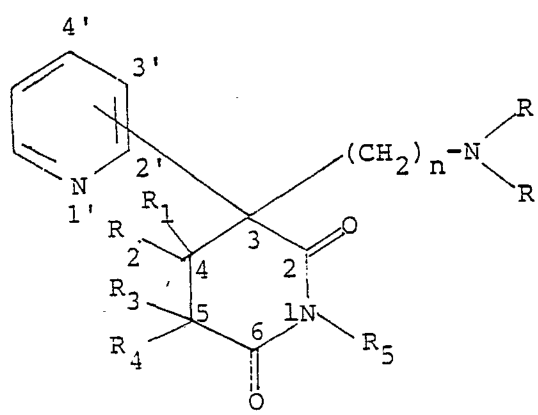

(I)

dans laquelle

R représente un groupe alkyle droit ou ramifié ayant 2 à 5 atomes de carbone, ou encore

— le groupe

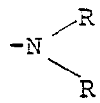

représente un groupe morpholino ou pipéridino éventuellement substitué par 1 à 4 groupes méthyle,

n = 2 ou 3,

$R_1$, $R_2$, $R_3$ et $R_4$ considérés indépendamment représentent un atome d'hydrogène ou un groupe alkyle droit ou ramifié ayant 1 à 4 atomes de carbone, ou

$R_3$ et $R_4$ pris ensemble représentent un groupe $(CH_2)_m$ où m = 4 ou 5, ou encore $R_2$ et $R_3$ pris ensemble représentent un groupe $(CH_2)_p$ où p = 3 ou 4 et, dans ce cas, $R_1$ et $R_4$ sont l'hydrogène,

$R_5$ désigne l'hydrogène ou un groupe alkyle droit ou ramifié ayant 1 à 4 atomes de carbone,

— enfin, la pipéridinedione substitue le groupe pyridyle en 2', 3' ou 4', ainsi que les sels desdits dérivés avec des acides pharmaceutiquement acceptables.

2. Dérivés selon la revendication 1, se présentant sous la forme d'un diastéréo-isomère ou d'un isomère optique ou d'un mélange de ces isomères.

3. Procédé de prépration des dérivés de la pipéridinedione selon la revendication 1, de formule I, dans laquelle $R_4 = R_5 = H$, caractérisé en ce que :

— dans une première étape, on réalise l'alkylation du pyridylacétonitrile par un composé de formule

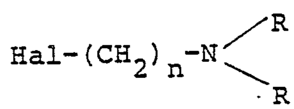

dans laquelle Hal représente un halogène, la réaction étant effectuée en présence d'une base,

— puis, on fait réagir sur le produit obtenu un nitrile α-insaturé de formule

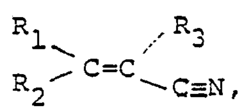

la réaction étant effectuée en présence d'un hydroxyde d'ammonium quaternaire,

— le composé ainsi obtenu est alors cyclisé par chauffage en milieu acide,

— et éventuellement transformé en l'un de ses sels par action d'un acide minéral ou organique pharmaceutiquement acceptable.

4. Procédé de préparation des dérivés de la pipéridinedione selon la revendication 1, de formule I, dans laquelle $R_1 = R_2 = R_5 = H$, caractérisé en ce que :

— dans une première étape, on fait régir un dérivé carbonylé de formule

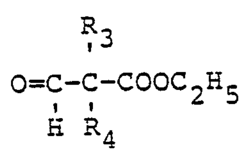 sur le pyridylacétonitrile,

— puis, on réduit catalytiquement le produit ainsi obtenu de façon à transformer le dérivé insaturé en dérivé saturé,

— puis, on fait réagir sur ledit dérivé saturé un α-nitrile de formule

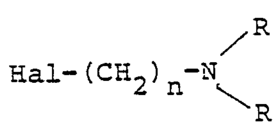

— le dérivé ainsi obtenu est ensuite cyclisé par chauffage en milieu acide,

— et éventuellement transformé en l'un de ses sels par action d'un acide minéral ou organique pharmaceutiquement acceptable.

5. Procédé de préparation des dérivés de la pipéridinedione selon la revendication 1, de formule I, dans laquelle $R_5 = H$,

caractérisé en ce que :

— dans une première étape, on effectue la réaction d'un nitrile insaturé de formule :

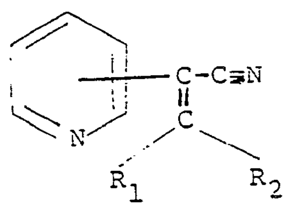

sur un nitrile de formule

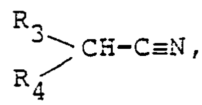

la réaction étant effectuée en présence de diisopropylamidure, à basse température et en solution dans un solvant,

— puis, on fait réagir le produit ainsi obtenu sur un dérivé halogéné de formule

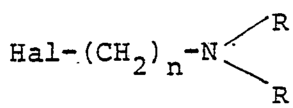

cette réaction étant effectuée en présence d'un agent de sodation au sein d'un solvant inerte,

— le produit ainsi obtenu est alors cyclisé par chauffage en milieu acide,

— et, éventuellement transformé en l'un de ses sels par action d'un acide minéral ou organique pharmaceutiquement acceptable.

6. Procédé de préparation des dérivés de la pipéridinedione selon la revendication 1, de formule I dans laquelle $R_1 = R_2 = R_5 = H$,
caractérisé en ce que :

— dans une première étape, on fait réagir le nitrile de formule

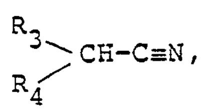

sur le chloro-1 bromo-2 éthane, la réaction étant réalisée en présence de diéthylamidure de lithium, à température ambiante, dans un solvant inerte,

— puis, on fait ragir le produit ainsi obtenu avec le cyanure de tétraéthylammonium à température de 40 à 60 °C, dans un solvant,

— le dinitrile ainsi obtenu est ensuite mis en réaction avec la bromopyridine, en présence de diisopropylamidure de lithium au sein d'un solvant inerte et à une température comprise entre — 10 et — 20 °C,

— on fait ensuite réagir le produit ainsi obtenu avec un dérivé halogéné de formule

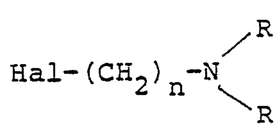

cette réaction étant effectuée en présence d'un agent de sodation au sein d'un solvant inerte,

— le produit ainsi obtenu est alors cyclisé par chauffage en milieu acide,

— et éventuellement transformé en l'un de ses sels par action d'un acide minéral ou organique pharmaceutiquement acceptable.

7. Procédé selon l'une quelconque des revendications 3 à 6, caractérisé en ce que le composé ainsi obtenu de formule I dans laquelle $R_5$ est H, avant une éventuelle salification, est transformé par alkylation à l'azote, selon des méthodes d'alkylation connues en un composé de formule I dans laquelle $R_5$ représente un groupe alkyle droit ou ramifié ayant 1 à 4 atomes de carbone.

8. Procédé selon l'une quelconque des revendications 3 à 7, caractérisé en ce que le composé de formule I ainsi obtenu est isolé sous la forme d'un diastéréo-isomère, d'un isomère optique ou d'un mélange de ces isomères.

9. Médicaments, caractérisés en ce qu'ils contiennent au moins un dérivé de la pipéridinedione selon l'une des revendications 1 ou 2.

10. Médicaments protecteurs du myocarde, caractérisés en ce qu'ils contiennent entre 50 et 800 mg d'au moins un dérivé de la pipéridinedione selon l'une des revendications 1 ou 2.

**Revendications** (pour l'Etat contractant AT)

1. Procédé de préparation de dérivés de la pipéridinedione de formule générale I :

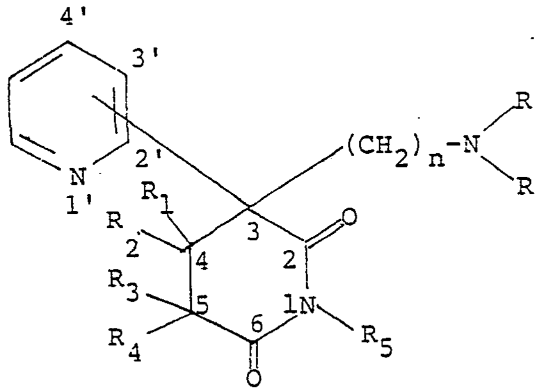

(I)

dans laquelle

R représente un groupe alkyle droit ou ramifié ayant 2 à 5 atomes de carbone, ou encore

— le groupe

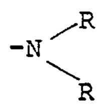

représente un groupe morpholino ou pipéridino éventuellement substitué par 1 à 4 groupes méthyle,

n = 2 ou 3,

$R_1$, $R_2$, $R_3$ considérés indépendamment représentent l'hydrogène ou un groupe alkyle droit ou ramifié ayant 1 à 4 atomes de carbone, ou $R_2$ et $R_3$ pris ensemble représentent $-(CH_2)_p-$ où p est 3 ou 4 et, dans ce cas, $R_1$ est H,

$R_4$ et $R_5$ sont H,

— la pipéridinedione substitue le groupe pyridyle en 2', 3'ou 4', ainsi que des sels desdits dérivés avec des acides,

caractérisé en ce que :

— dans une première étape, on réalise l'alkylation du pyridylacétonitrile par un composé de formule

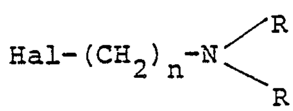

dans laquelle Hal représente un halogène, la réaction étant effectuée en présence d'une base,

— puis, on fait réagir sur le produit ainsi obtenu un nitrile α-insaturé de formule

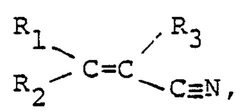

la réaction étant effectuée en présence d'un hydroxyde d'ammonium quaternaire,

— le composé ainsi obtenu est alors cyclisé par chauffage en milieu acide,

— éventuellement, on transforme le produit de formule I ainsi obtenu en l'un de ses sels par action d'un acide organique ou minéral.

2. Procédé de préparation de dérivés de la pipéridinedione de formule générale I :

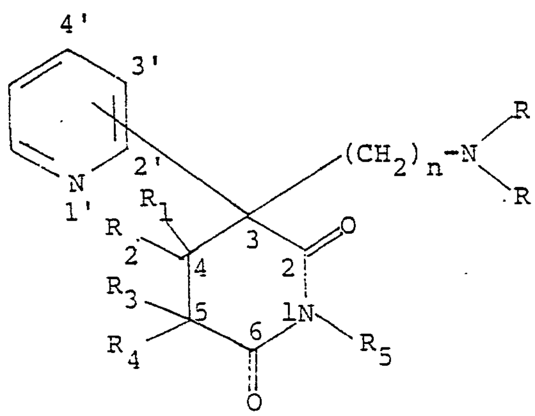

(I)

dans laquelle

R représente un groupe alkyle droit ou ramifié ayant 2 à 5 atomes de carbone, ou encore

— le groupe

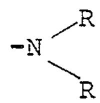

représente un groupe morpholino ou pipéridino éventuellement substitué par 1 à 4 groupes méthyle,

n = 2 ou 3,

$R_1$, $R_2$, $R_5$ sont H,

$R_3$ et $R_4$, considérés indépendamment, représentent l'hydrogène ou un groupe alkyle droit ou ramifié ayant 1 à 4 atomes de carbone, ou $R_3$ et $R_4$ pris ensemble représentent un groupe $(CH_2)_m$ où m = 4 ou 5,

— la pipéridinedione substitue le groupe pyridyle en 2', 3' ou 4', ainsi que des sels desdits dérivés avec des acides,

caractérisé en ce que :

— dans une première étape, on fait réagir un dérivé carbonylé de formule

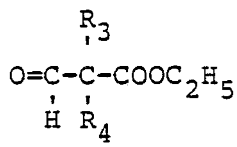

sur le pyridylacétonitrile,

— puis, on réduit catalytiquement le produit ainsi obtenu de façon à transformer le dérivé insaturé en dérivé saturé,

— puis, on fait réagir sur le dérivé saturé un α-nitrile de formule

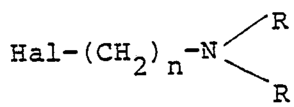

— le dérivé ainsi obtenu est ensuite cyclisé par chauffage en milieu acide,

— et éventuellement, on transforme le produit de formule I ainsi obtenu en l'un de ses sels par action d'un acide minéral ou organique.

3. Procédé de préparation de dérivés de la pipéridinedione de formule générale I :

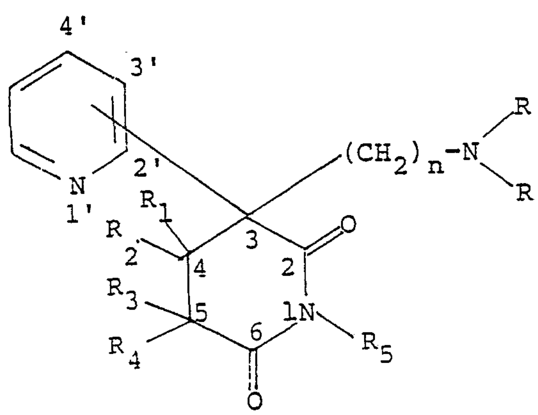

(I)

dans laquelle

R représente un groupe alkyle droit ou ramifié ayant 2 à 5 atomes de carbone, ou encore

— le groupe

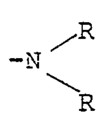

représente un groupe morpholino ou pipéridino éventuellement substitué par 1 à 4 groupes méthyle,

n = 2 ou 3,

$R_1$, $R_2$, $R_3$ et $R_4$ considérés indépendamment représentent un atome d'hydrogène ou un groupe alkyle droit ou ramifié ayant 1 à 4 atomes de carbone, ou

$R_3$ et $R_4$ pris ensemble représentent un groupe $(CH_2)_m$ où m = 4 ou 5, ou encore $R_2$ et $R_3$ pris ensemble représente un groupe $(CH_2)_p$ où p = 3 ou 4 et, dans ce cas, $R_1$ et $R_4$ sont l'hydrogène,

$R_5$ est H,

— la pipéridinedione substitue le groupe pyridyle en 2', 3' ou 4', ainsi que des sels desdits dérivés avec des acides,

caractérisé en ce que :

— dans une première étape on effectue la réaction d'un nitrile insaturé de formule

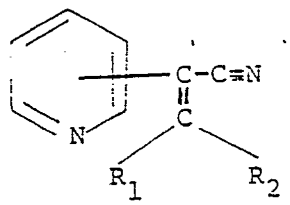

sur un nitrile de formule

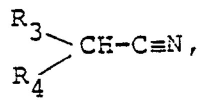

la réaction étant effectuée en présence de diisopropylamidure, à basse température et en solution dans un solvant,

— puis, on fait réagir le produit ainsi obtenu sur un dérivé halogéné de formule

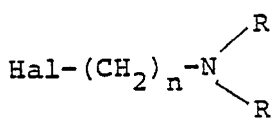

cette réaction étant effectuée en présence d'un agent de sodation au sein d'un solvant inerte,

— puis, on cyclise par chauffage en milieu acide le produit ainsi obtenu,

— éventuellement, on transforme le produit de formule I ainsi obtenu en l'un de ses sels par action d'un acide minéral ou organique.

4. Procédé de préparation de dérivés de la pipéridinedione de formule générale I :

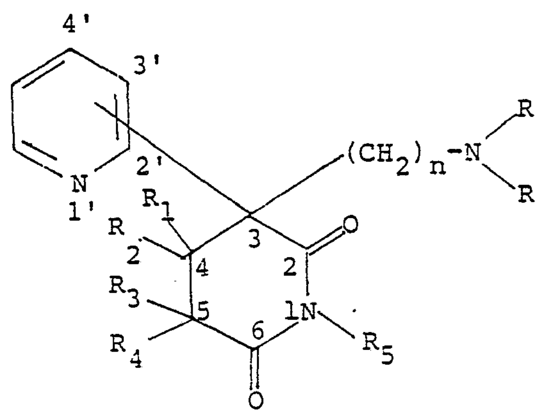

(I)

dans laquelle

R représente un groupe alkyle droit ou ramifié ayant 2 à 5 atomes de carbone, ou encore,

— le groupe

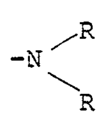

représente un groupe morpholino ou pipéridino éventuellement substitué par 1 à 4 groupes méthyle,

n = 2 ou 3,

$R_1$, $R_2$ et $R_5$ sont H,

$R_3$ et $R_4$ considérés indépendamment représentent l'hydrogène ou un groupe alkyle droit ou ramifié ayant 1 à 4 atomes de carbone ou $R_3$ et $R_4$ pris ensemble représentent un groupe $(CH_2)_m$ où m = 4 ou 5,

— la pipéridinedione substitue le groupe pyridyle en 2', 3' ou 4', ainsi que des sels desdits dérivés avec des acides,

caractérisé en ce que :

— dans une première étape, on fait réagir le nitrile de formule

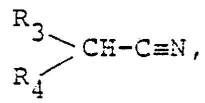

sur le chloro-1 bromo-2 éthane, la réaction étant réalisée en présence de diéthylamidure de lithium, à température ambiante, dans un solvant inerte,

— puis, on fait réagir le produit ainsi obtenu avec le cyanure de tétraéthylammonium à température de 40 à 60 °C, dans un solvant,

— le dinitrile ainsi obtenu est ensuite mis en réaction avec la bromopyridine, en présence de diisopropylamidure de lithium au sein d'un solvant inerte et à une température comprise entre — 10 et — 20 °C,

— on fait ensuite réagir le produit obtenu avec un dérivé halogéné de formule

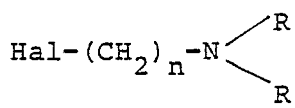

cette réaction étant effectuée en présence d'un agent de sodation au sein d'un solvant inerte,

— puis, on cyclise par chauffage en milieu acide le produit ainsi obtenu,

— éventuellement, on transforme le produit de formule I ainsi obtenu en l'un de ses sels par action d'un acide minéral ou organique.

5. Procédé de préparation de dérivés de la pipéridinedione de formule I selon l'une quelconque des revendications 1 à 4, caractérisé en ce que ledit dérivé de formule I dans laquelle $R_5$ est H, avant une éventuelle salification, est transformé par alkylation à l'azote, selon des méthodes d'alkylation connues, en un composé de formule I dans laquelle $R_5$ représente un groupe alkyle droit ou ramifié ayant de 1 à 4 atomes de carbone.

6. Procédé selon l'une quelconque des revendications 1 à 5, caractérisé en ce que le produit ainsi obtenu est isolé sous la forme d'un diastéréo-isomère, d'un isomère optique ou d'un mélange de ces isomères.

7. Utilisation des dérivés de la pipéridinedione de formule I, obtenus selon l'une quelconque des revendications 1 à 6, pour la préparation de médicaments.

8. Utilisation de dérivés de la pipéridinedione de formule I, obtenus selon l'une quelconque des revendications 1 à 6, pour la préparation de médicaments protecteurs du myocarde, lesdits médicaments contenant entre 50 et 800 mg desdits dérivés.

**Claims** (for the Contracting States : BE, CH, DE, FR, GB, IT, LI, LU, NL, SE)

1. Derivatives of piperidinedione responding to general formula I :

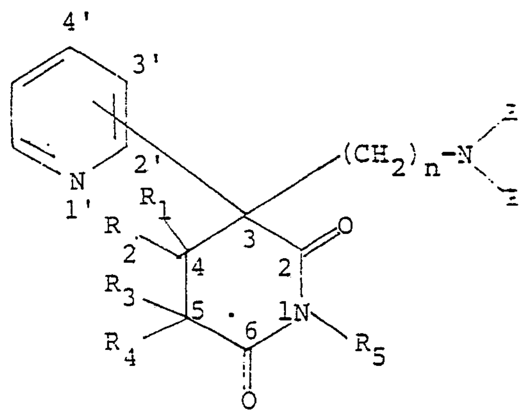

(I)

in which

R represents a straight or branched alkyl group having from 2 to 5 atoms of carbon, or the group

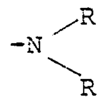

represents a morpholino or piperidino group possibly substituted by 1 to 4 methyl groups ;

$n = 2$ or 3 ;

$R_1$, $R_2$, $R_3$ and $R_4$, considered independently represent an atom of hydrogen or a straight or branched alkyl group having 1 to 4 atoms of carbon, or

$R_3$ and $R_4$ taken together represent a $(CH_2)_m$ group where $m = 4$ or 5 or $R_2$ and $R_3$ taken together represent a $(CH_2)_p$ group where $p = 3$ or 4 and in this case $R_1$ and $R_4$ are hydrogen,

$R_5$ designates hydrogen or a straight or branched alkyl group having 1 to 4 atoms of carbon,

— finally piperidinedione substitutes the pyridyl group in 2', 3' or 4' position, as well as the salts of said derivatives with pharmaceutically acceptable acids.

2. The derivatives according to claim 1 in the form of a diastereoisomer or an optical isomer or a mixture of these isomers.

3. A process for preparing the derivatives of piperidinedione of formula I, according to claim 1, in which $R_4 = R_5 = H$, characterized in that it comprises :

— in a first step, effecting alkylation of pyridylacetonitrile by a compound of formula

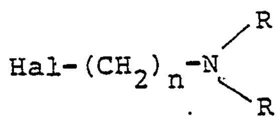

in which Hal represents a halogen, the reaction being carried out in the presence of a base,

— then reacting on the product obtained an α-unsaturated nitrile of formula

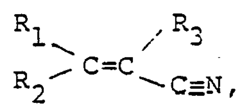

the reaction being carried out in the presence of a quaternary ammonium hydroxide,

— then cyclizing the compound obtained by heating in an acid medium,

— and optionally converting the product into one of its salts by action of a pharmaceutically acceptable mineral or organic acid.

4. A process for preparing the derivatives of piperidinedione of formula (I) according to claim 1, in which $R_1 = R_2 = R_5 = H$, characterized in that it comprises :

— in a first step, reacting a carbonyl derivative of formula :

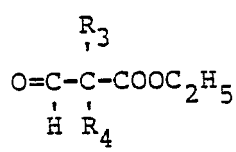 on pyridylacetonitrile,

— then catalytically reducing the product thus obtained so as to convert the unsaturated derivative into saturated derivative,

— then reacting on said saturated derivative an α-nitrile of formula

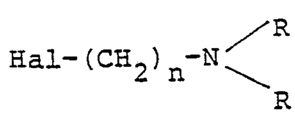

— then cyclizing the derivative obtained by heating in an acid medium.

— and optionally converting the derivative into one of its salts by action of a pharmaceutically acceptable mineral or organic acid.

5. A process for preparing the derivatives of piperidinedione of formula (I) according to claim 1, in which $R_5 = H$, characterized in that it comprises :

— in first step, effecting reaction of an unsaturated nitrile of formula

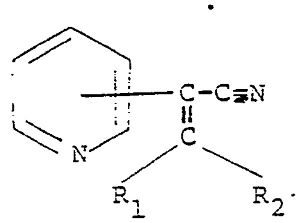

on an nitrile of formula

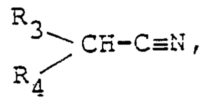

the reaction being effected in the presence of diisopropylamide, at low temperature and in solution in a solvent,

— then reacting the product thus obtained on a halogen derivative of formula

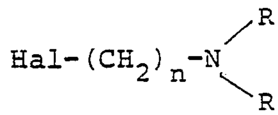

this reaction being carried out in the presence of a sodation agent within an inert solvent,

— then cyclizing the product obtained by heating in an acid medium,

— and optionally converting the product into one of its salt by action of a pharmaceutically acceptable mineral or organic acid.

6. A process for preparing the derivatives of piperidinedione of formula (I) according to claim 1, in which $R_1 = R_2 = R_5 = H$, characterized in that it comprises :

— in a first step, reacting the nitrile of formula

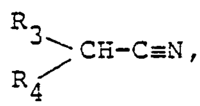

on 1-chloro 2-bromo ethane, the reaction being carried out in the presence of lithium diethylamide, at ambient temperature, in an inert solvent,

— then reacting the product thus obtained with tetraethylammonium cyanide at a temperature of 40 to 60°, in a solvent,

— then reacting the dinitrile obtained with bromopyridine, in the presence of lithium diisopropylamide within an inert solvent and at a temperature of between —10 and —20 °C,

— then reacting the product thus obtained with a halogen derivative of formula

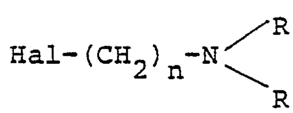

this reaction being carried out in the presence of a sodation agent within an inert solvent.

— then cyclizing the product thus obtained by heating in an acid medium,

— and optionally converting the product into one of its salts by action of a pharmaceutically acceptable mineral or organic acid.

7. The process according to any one of claims 3 to 6, characterized in that the product thus obtained of formula I in which $R_5$ is H, is converted, before an optional salification, by alkylation with nitrogen, in accordance with known methods of alkylation into a compound of formula I in which $R_5$ represents a straight or branched alkyl group having 1 to 4 atoms of carbon.

8. The process according to any one of claims 3 to 7, characterized in that the product thus obtained of formula I is isolated in the form of a diastereo-isomer, of an optical isomer or of a mixture of these isomers.

9. Drugs characterized in that they contain at least one derivative of piperidinedione according to any one of claims 1 or 2.

10. Drugs protecting the myocardium, characterized in that they contain between 50 and 800 mg of at least one derivative of piperidinedione according to any one of claims 1 or 2.

**Claims** (for the Contracting State AT)

1. Process for preparing derivatives of piperidinedione responding to the general formula I :

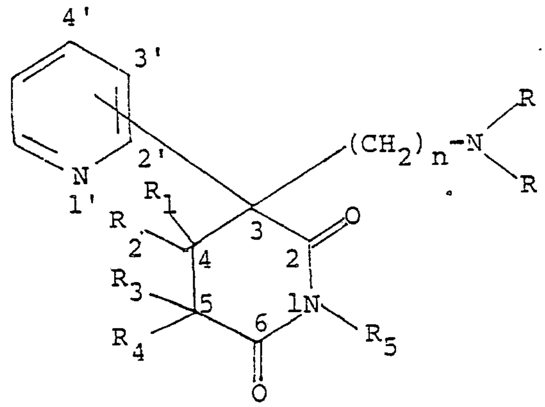

(I)

in which

R represents a straight or branched alkyl group having from 2 to 5 atoms of carbon,

— or the group

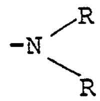

represents a morpholino or piperidino group possibly substituted by 1 to 4 methyl groups,

n = 2 or 3,

$R_1$, $R_2$, $R_3$ considered independently represent hydrogen or a straight or branched alkyl group having 1 to 4 atoms of carbon, or $R_2$ and $R_3$ taken together represent $-(CH_2)_p-$ where p = 3 or 4, and in this case $R_1$ = H,

$R_4$ and $R_5$ = H,

— the piperidinedione substitutes the pyridyl group in 2′, 3′ or 4′ position, as well as the salts of said derivatives with acids, characterized in that it comprises :

— in a first step, effecting alkylation of pyridylacetonitrile by a compound of formula

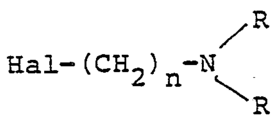

in which Hal represents a halogen, the reaction being carried out in the presence of a base,

— then reacting on the product thus obtained an α-unsaturated nitrile of formula

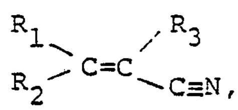

the reaction being carried out in the presence of a quaternary ammonium hydroxide,

— then cyclizing the product thus obtained by heating in an acid medium,

— and optionally converting the product thus obtained, of formula I, into one of its salts by action of a mineral or organic acid.

2. Process for preparing derivatives of piperidinedione of general formula I :

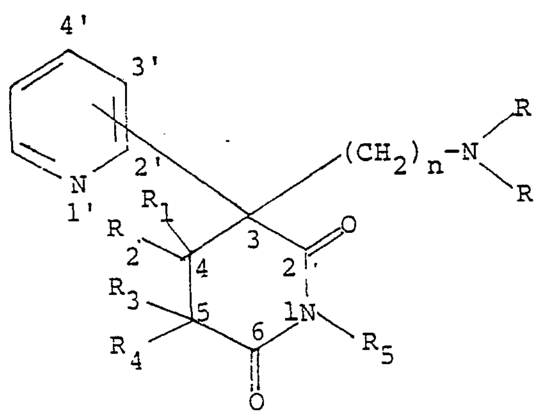

(I)

in which

R represents a straight or branched alkyl group having from 2 to 5 atoms of carbon, or — the

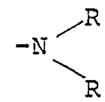

group represents a morpholino or piperidino group possibly substituted by 1 to 4 methyl groups,

n = 2 or 3,

$R_1$, $R_2$, $R_5$ = H,

$R_3$ and $R_4$, considered independently represent hydrogen or a straight or branched alkyl group having from 1 to 4 atoms of carbon, or $R_3$ and $R_4$ taken together represent a $(CH_2)_m$ group where m = 4 or 5,

— the piperidinedione substitutes the pyridyl group in 2', 3' or 4' position, as well as the salts of said derivatives with the acids,

characterized in that it comprises :

— in a first step, reacting a carbonyl derivative of formula

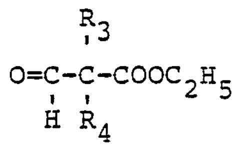 on pyridylacetonitrile,

— then catalytically reducing the product thus obtained so as to convert the unsaturated derivative into saturated derivative,

— then reacting on said saturated derivative a α-nitrile of formula

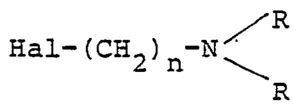

— then cyclizing the derivative thus obtained by heating in an acid medium,

— and optionally converting the product of formula I thus obtained into one of its salts by action of a mineral or organic acid.

3. Process for preparing derivatives of piperidinedione of general formula I :

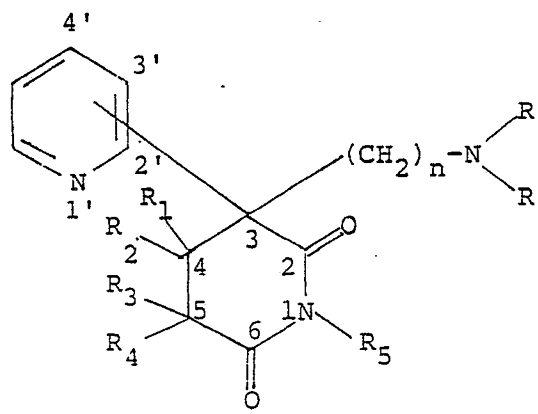

(I)

in which

R represents a straight or branched alkyl group having from 2 to 5 atoms of carbon, or — the

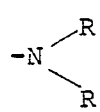

group represents a morpholino or piperidino group possibly substituted by 1 to 4 methyl groups,

n = 2 or 3,

$R_1$, $R_2$, $R_3$ and $R_4$ considered independently represent an atom of hydrogen or a straight or branched alkyl group having from 1 to 4 toms of carbon, or

$R_3$ and $R_4$ taken together represent a $(CH_2)_m$ group where m = 4 or 5, or $R_2$ and $R_3$ taken together represent a $(CH_2)_p$ group where p = 3 or 4, and in this case $R_1$ and $R_4$ are hydrogen ;

$R_5$ is H,

— the piperidinedione substitutes the pyridyl group in 2', 3' or 4' position, as well as the salts of said derivatives with acids,

characterized in that it comprises :

— in a first step, effecting reaction of an unsaturated nitrile of formula

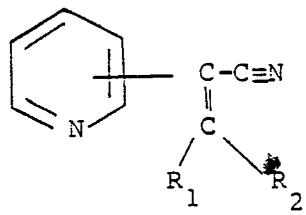

on a nitrile of formula

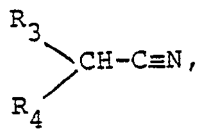

the reaction being effected in the presence of diisopropylamide, at low temperature and in solution in a solvent,

— then reacting the product thus obtained on a halogen derivative of formula

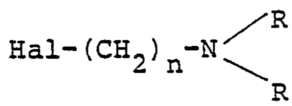

this reaction being carried out in the presence of a sodation agent within an inert solvent,

— then cyclizing the product thus obtained by heating in an acid medium,

— and optionally converting the product thus obtained of formula I into one of its sals by action of a mineral or organic acid.

4. Process for preparing derivatives of piperidinedione of general formula I :

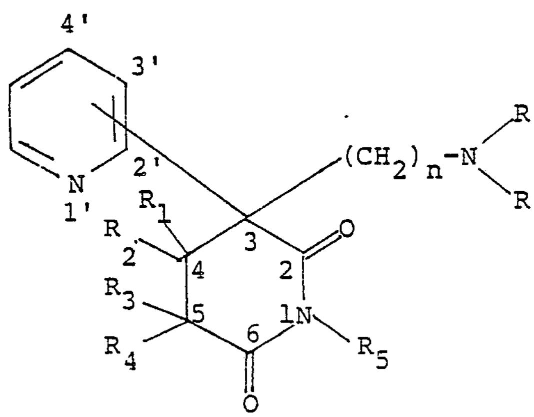

(I)

in which

R represents a straight or branched alkyl group having from 2 to 5 atoms of carbon, or

— the

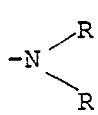

group represents a morpholino or piperidino group possibly substituted by 1 to 4 methyl groups,

n = 2 or 3,

$R_1$, $R_2$ and $R_5$ = H,

$R_3$ and $R_4$ considered independently represent hydrogen or a straight or branched alkyl group having from 1 to 4 atoms of carbon, or

$R_3$ and $R_4$ taken together represent a $(CH_2)_m$ group where m = 4 or 5,

— the piperidinedione substitutes the pyridyl group in 2', 3' or 4' position, as well as the salts of said derivatives with acids,

characterized in that it comprises :

— in a first step, reacting the nitrile of formula

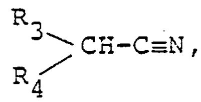

on 1-chloro 2-bromo ethane, the reaction being carried out in the presence of lithium diethylamide, at ambient temperature, in an inert solvent,

— then reacting the product thus obtained with tetraethylammonium cyanide at a temperature of 40 to 60°, in a solvent,

— then reacting the dinitrile obtained with bromopyridine, in the presence of lithium diisopropylamide within an inert solvent and at a temperature of between —10 and —20 °C,

— then reacting the product obtained with a halogen derivative of formula

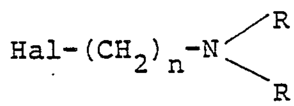

this reaction being carried out in the presence of a sodation agent within an inert solvent,

— then cyclizing the product thus obtained by heating in an acid medium,

— and optionally converting the product into one of its salts by action of a mineral or organic acid.

5. Process for preparing derivatives of piperidinedione of formula I according to any one of claims 1 to 4, characterized in that said derivative of formula I in which $R_5$ is H, is converted before a possible salification, by alkylation with nitrogen, in accordance with known methods of alkylation into a compound of formula I in which $R_5$ represents a straight or branched alkyl group having from 1 to 4 atoms of carbon.

6. Process according to any one of claims 1 to 5, characterized in that the product thus obtained is isolated in the form of a diastereoisomer, of an optical isomer or of a mixture of these isomers.

7. Use of derivatives of piperidinedione of formula I, obtained according to any one of claims 1 to 6, for the preparation of drugs.

8. Use of derivatives of piperidinedione of formula I, obtained according to any one of claims 1 to 6, for the preparation of drugs protecting the myocardium, said drugs containing between 50 and 800 mg of said derivatives.

**Patentansprüche** (für die Vertragsstaaten : BE, CH, DE, FR, GB, IT, LI, LU, NL, SE)

1. Piperidindionderivate der allgemeinen Formel I

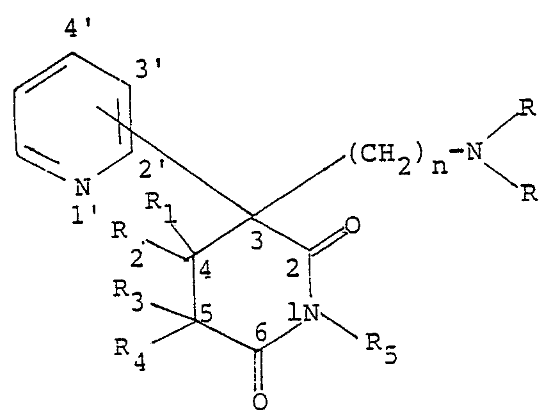

(I)

worin

R für eine gerade oder verzweigte Alkylgruppe mit 2 bis 5 Kohlenstoffatomen steht, oder aber

— die Gruppe

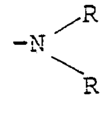

eine gegebenenfalls durch 1 bis 4 Methylgruppen substituierte Morpholino- oder Piperidinogruppe darstellt,

n = 2 oder 3,

$R_1$, $R_2$, $R_3$ und $R_4$, unabhängig voneinander, ein Wasserstoffatom oder eine gerade oder verzweigte Alkylgruppe mit 1 bis 4 Kohlenstoffatomen darstellen, oder

$R_3$ und $R_4$, zusammen genommen, eine Gruppe $(CH_2)_m$ bedeuten, worin m = 4 oder 5, oder aber $R_2$ und $R_3$, zusammen genommen, eine Gruppe $(CH_2)_p$ darstellen, worin p = 3 oder 4, und in diesem Fall $R_1$ und $R_4$ Wasserstoff sind,

$R_5$ Wasserstoff oder eine gerade oder verzweigte Alkylgruppe mit 1 bis 4 Kohlenstoffatomen bezeichnet,

— und schließlich das Piperidindion die Pyridylgruppe in 2', 3' oder 4' substituiert,

sowie die Salze der Derivate mit pharmazeutisch akzeptablen Säuren.

2. Derivate nach Anspruch 1 in Form eines Diastereoisomeren oder optischen Isomeren oder einer Mischung dieser Isomeren.

3. Verfahren zur Herstellung von Piperidindionderivaten nach Anspruch 1 der Formel I, worin $R_4 = R_5 = H$, dadurch gekennzeichnet, daß

— in einer ersten Stufe die Alkylierung von Pyridylacetonitril mit einer Verbindung der Formel

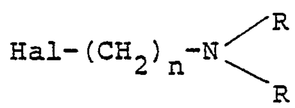

worin Hal für ein Halogen steht, durchgeführt wird, wobei die Reaktion in Gegenwart einer Base erfolgt,

— dann auf das erhaltene Produkt ein α-ungesättigtes Nitril der Formel

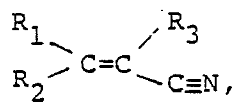

einwirken gelassen wird, wobei die Reaktion in Gegenwart eines quaternären Ammoniumhydroxids ausgeführt wird,

— die so erhaltene Verbindung danach durch Erhitzen in saurem Milieu zyklisiert wird,

— und gegebenenfalls durch Einwirken einer pharmazeutisch akzeptablen Mineral- oder organischen Säure in eines ihrer Salze übergeführt wird.

4. Verfahren zur Herstellung von Piperidindionderivaten nach Anspruch 1 der Formel I, worin $R_1 = R_2 = R_5 = H$, dadurch gekennzeichnet, daß:

— in einer ersten Stufe ein Carbonylderivat der Formel

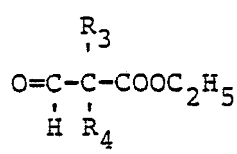

mit Pyridylacetonitril zur Umsetzung gebracht wird,

— dann das so erhaltene Produkt katalytisch reduziert wird, sodaß das ungesättigte Derivat in ein gesättigtes Derivat übergeführt wird,

— dann auf das gesättigte Derivat ein α-Nitril der Formel

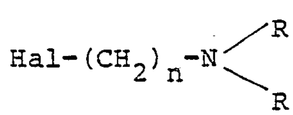

einwirken gelassen wird,

— das so erhaltene Derivat danach durch Erhitzen in saurem Milieu zyklisiert wird,

— und gegebenenfalls durch Einwirken einer pharmazeutisch akzeptablen Mineral- oder organischen Säure in eines seiner Salze übergeführt wird.

5. Verfahren zur Herstellung von Piperidindionderivaten nach Anspruch 1 der Formel I, worin $R_5 = H$, dadurch gekennzeichnet, daß

— in einer ersten Stufe die Umsetzung eines ungesättigten Nitrils der Formel

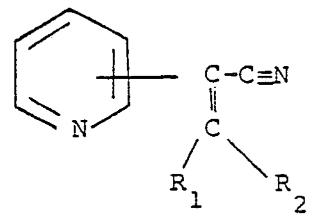

mit einem Nitril der Formel

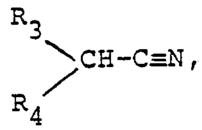

durchgeführt wird, wobei die Reaktion in Gegenwart eines Metall-Diisopropylamids bei niedriger Temperatur und in Lösung in einem Lösungsmittel erfolgt,

— dann das so erhaltene Produkt mit einem halogenierten Derivat der Formel

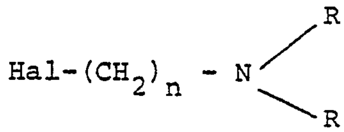

zur Umsetzung gebracht wird, wobei diese Reaktion in Gegenwart eines natrisiernden Mittels in einem inerten Lösungsmittel durchgeführt wird,

— das so erhaltene Produkt danach durch Erhitzen in saurem Milieu zyklisiert wird,

— und gegebenenfalls durch Einwirken einer pharmazeutisch akzeptablen Mineral- oder organischen Säure in eines seiner Salze übergeführt wird.

6. Verfahren zur Herstellung von Piperidindionderivaten nach Anspruch 1 der Formel I, worin $R_1 = R_2 = R_5 = H$, dadurch gekennzeichnet, daß

— in einer ersten Stufe das Nitril der Formel

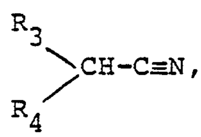

mit 1-Chlor-2-brom-äthan zur Umsetzung gebracht wird, wobei die Reaktion in Gegenwart von Lithiumdiäthylamid bei Umgebungstemperatur in einem inerten Lösungsmittel durchgeführt wird,

— und dann das so erhaltene Produkt mit Tetraäthylammoniumcyanid bei einer Temperatur von 40 bis 60 °C in einem Lösungsmittel reagieren gelassen wird,

— das so erhaltene Dinitril danach mit Brompyridin in Gegenwart von Lithiumdiisopropylamid in einem inerten Lösungsmittel und bei einer Temperatur zwischen —10 und —20 °C zur Umsetzung gebracht wird,

— danach das so erhaltene Produkt mit einem halogenierten Derivat der Formel

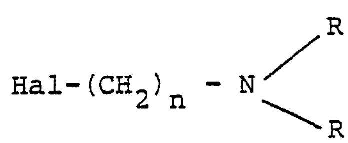

reagieren gelassen wird, wobei die Reaktion in Gegenwart eines natrisierenden Mittels in einem inerten Lösungsmittel durchgeführt wird,

— das so erhaltene Produkt danach durch Erhitzen in saurem Milieu zyklisiert wird,

— und gegebenenfalls durch Einwirken einer pharmazeutisch akzeptablen Mineral- oder organischen Säure in eines seiner Salze übergeführt wird.

7. Verfahren nach einem der Ansprüche 3 bis 6, dadurch gekennzeichnet, daß die so erhaltene Verbindung der Formel I, worin $R_5$ für H steht, vor einer möglichen Salzbildung durch Alkylieren am Stickstoff nach bekannten Alkylierungsverfahren in eine Verbindung der Formel I, worin $R_5$ eine gerade oder verzweigte Alkylgruppe mit 1 bis 4 Kohlenstoffatomen darstellt, übergeführt wird.

8. Verfahren nach einem der Ansprüche 3 bis 7, dadurch gekennzeichnet, daß die so erhaltene Verbindung der Formel I in Form eines Diastereoisomeren, eines optischen Isomeren oder einer Mischung dieser Isomeren isoliert wird.

9. Medikamente, dadurch gekennzeichnet, daß sie mindestens ein Piperidindionderivat nach einem der Ansprüche 1 oder 2 enthalten.

10. Medikamente zum Myokardschutz, dadurch gekennzeichnet, daß sie zwischen 50 und 800 mg mindestens eines Piperidindionderivats nach einem der Ansprüche 1 oder 2 enthalten.

**Patentansprüche** (für den Vertragsstaat AT)

1. Verfahren zur Herstellung von Piperidindionderivaten der allgemeinen Formel I

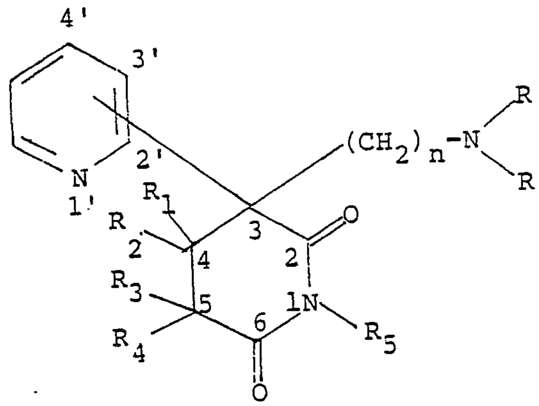

(I)

worin

R für eine gerade oder verzweigte Alkylgruppe mit 2 bis 5 Kohlenstofftomen steht, oder aber

— die Gruppe

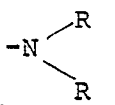

eine gegebenenfalls durch 1 bis 4 Methylgruppen substituierte Morpholino- oder Piperidinogruppe darstellt,

n = 2 oder 3,

$R_1$, $R_2$, $R_3$, unabhängig voneinander Wasserstoff oder eine gerade oder verzweigte Alkylgruppe mit 1 bis 4 Kohlenstoffatomen darstellen, oder $R_2$ und $R_3$, zusammen genommen, für $-(CH_2)_p-$ stehen, worin p 3 oder 4 ist, und in diesem Fall $R_1$ für H steht,

$R_4$ und $R_5$ H bedeuten,

— das Piperidindion die Pyridylgruppe in 2', 3' oder 4' substituiert,

sowie der Salze dieser Derivate mit Säuren, dadurch gekenzeichnet, daß

— in einer ersten Stufe die Alkylierung von Pyridylacetonitril mit einer Verbindung der Formel

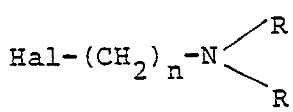

worin Hal für ein Halogen steht, durchgeführt wird, wobei die Reaktion in Gegenwart einer Base erfolgt,

— dann auf das erhaltene Produkt ein α-ungesättigtes Nitril der Formel

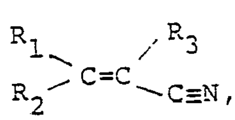

einwirken gelassen wird, wobei die Reaktion in Gegenwart eines quaternären Ammoniumhydroxids ausgeführt wird,

— die so erhaltene Verbindung danach durch Erhitzen in saurem Milieu zyklisiert wird,

— gegebenenfalls das so erhaltene Produkt der Formel I durch Einwirken einer organischen oder Mineralsäure in eines seiner Salze übergeführt wird.

2. Verfahren zur Herstellung von Piperidindionderivaten der allgemeinen Formel I

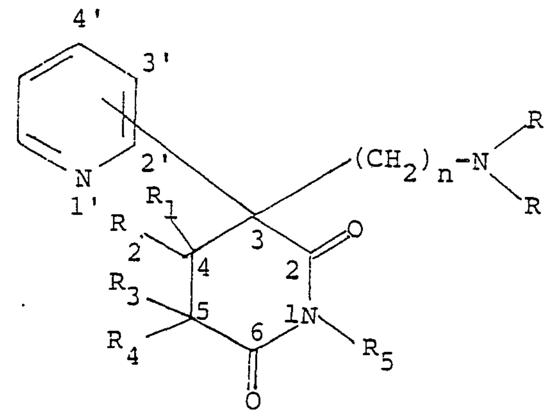

(I)

worin

R für eine gerade oder verzweigte Alkylgruppe mit 2 bis 5 Kohlenstoffatomen steht, oder aber — die Gruppe

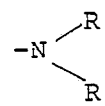

eine gegebenenfalls durch 1 bis 4 Methylgruppen substituierte Morpholino- oder Piperidinogruppe darstellt,

n = 2 oder 3,

$R_1$, $R_2$, $R_5$ H bedeuten,

$R_3$ und $R_4$, unabhängig voneinander, Wasserstoff oder eine gerade oder verzweigte Alkylgruppe mit 1 bis 4 Kohlenstoffatomen darstellen, oder $R_3$ und $R_4$, zusammen genommen, eine Gruppe $(CH_2)_m$ bedeuten, worin m = 4 oder 5,

— das Piperidindion die Pyridylgruppe in 2', 3' oder 4' substituiert,

sowie der Salze dieser Derivate mit Säuren, dadurch gekennzeichnet, daß :

— in einer ersten Stufe ein Carbonylderivat der Formel

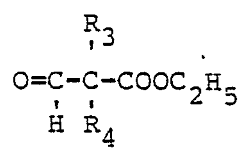

mit Pyridylacetonitril zur Umsetzung gebracht wird,

— dann das so erhaltene Produkt katalytisch reduziert wird, sodaß das ungesättigte Derivat in ein gesättigtes Derivat übergeführt wird,

— dann auf das gesättigte Derivat ein α-Nitril der Formel

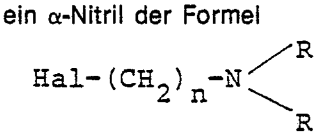

einwirken gelassen wird,

— das so erhaltene Derivat danach durch Erhitzen in saurem Milieu zyklisiert wird,

— und gegebenenfalls das so erhaltene Produkt der Formel I durch Einwirken einer Mineral- oder organischen Säure in eines seiner Salze übergeführt wird.

3. Verfahren zur Herstellung von Piperidindionderivaten der allgemeinen Formel I

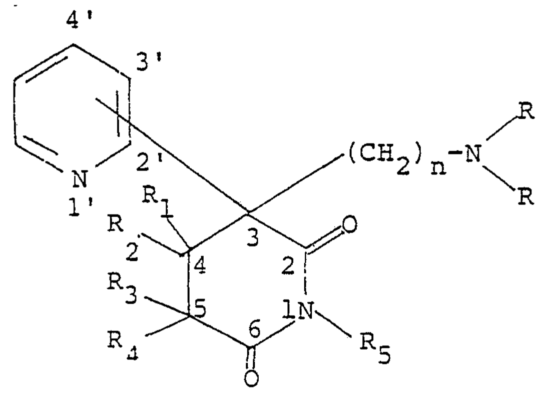

(I)

worin

R für eine gerade oder verzweigte Alkylgruppe mit 2 bis 5 Kohlenstoffatomen steht, oder aber

— die Gruppe

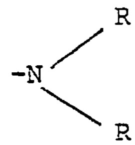

eine gegebenenfalls durch 1 bis 4 Methylgruppen substituierte Morpholino- oder Piperidinogruppe darstellt,

n = 2 oder 3,

$R_1$, $R_2$, $R_3$ und $R_4$, unabhängig voneinander, ein Wasserstoffatom oder eine gerade oder verzweigte Alkylgruppe mit 1 bis 4 Kohlenstoffatomen darstellen, oder

$R_3$ und $R_4$, zusammen genommen, eine Gruppe $(CH_2)_m$ bedeuten, worin m = 4 oder 5, oder aber $R_2$ und $R_3$, zusammen genommen, eine Gruppe $(CH_2)_p$ darstellen, worin p = 3 oder 4, und in diesem Fall $R_1$ und $R_4$ Wasserstoff sind,

$R_5$ für H steht,

— das Piperidindion die Pyridylgruppe in 2', 3' oder 4' substituiert,

sowie der Salze dieser Derivate mit Säuren, dadurch gekennzeichnet, daß

— in einer ersten Stufe die Umsetzung eines ungesättigten Nitrils der Formel

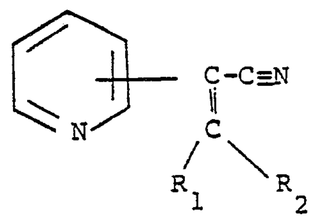

mit einem Nitril der Formel

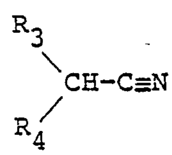

durchgeführt wird, wobei die Reaktion in Gegenwart eines Metall-Diisopropylamids bei niedriger Temperatur und in Lösung in einem Lösungsmittel erfolgt,

— dann das so erhaltene Produkt mit einem halogenierten Derivat der Formel

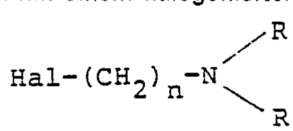

zur Umsetzung gebracht wird, wobei diese Reaktion in Gegenwart eines natrisierenden Mittels in einem inerten Lösungsmittel durchgeführt wird,

— das so erhaltene Produkt danach durch Erhitzen in saurem Milieu zyklisiert wird,

— gegebenenfalls das so erhaltene Produkt der Formel I durch Einwirken einer Mineral- oder organischen Säure in eines seiner Salze übergeführt wird.

4. Verfahren zur Herstellung von Piperidindionderivaten der allgemeinen Formel I

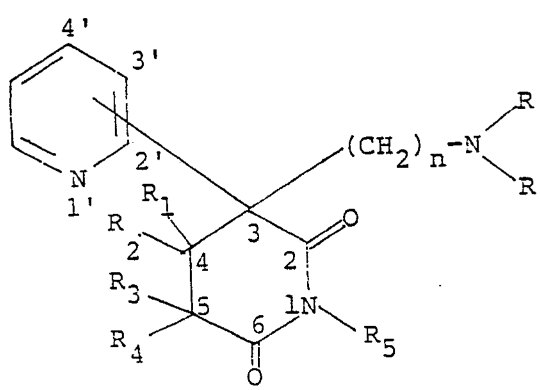

(I)

worin

R für eine gerade oder verzweigte Alkylgruppe mit 2 bis 5 Kohlenstoffatomen steht, oder aber

— die Gruppe

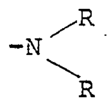

eine gegebenenfalls durch 1 bis 4 Methylgruppen substituierte Morpholino- oder Piperidinogruppe darstellt,

n = 2 oder 3,

$R_1$, $R_2$ und $R_5$ H bedeuten,

$R_3$ und $R_4$, unabhängig voneinander, Wasserstoff oder eine gerade oder verzweigte Alkylgruppe mit 1 bis 4 Kohlenstoffatomen bedeuten oder

$R_3$ und $R_4$, zusammen genommen, eine Gruppe $(CH_2)_m$ darstellen, worin m = 4 oder 5,

— das Piperidindion die Pyridylgruppe in 2', 3' oder 4' substituiert,

sowie der Salze dieser Derivate mit Säuren, dadurch gekennzeichnet, daß

— in einer ersten Stufe das Nitril der Formel

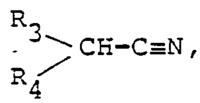

mit 1-Chlor-2-brom-äthan zur Umsetzung gebracht wird, wobei die Reaktion in Gegenwart von Lithiumdiäthylamid bei Umgebungstemperatur in einem inerten Lösungsmittel durchgeführt wird,

— dann das so erhaltene Produkt mit Tetraäthylammoniumcyanid bei einer Temperatur von 40 bis 60 °C in einem Lösungsmittel reagieren gelassen wird,

— das so erhaltene Dinitril danach mit Brompyridin in Gegenwart von Lithiumdiisopropylamid in einem inerten Lösungsmittel und bei einer Temperatur zwischen —10 und —20 °C zur Umsetzung gebracht wird,

— danach das so erhaltene Produkt mit einem halogenierten Derivat der Formel

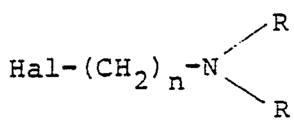

reagieren gelassen wird, wobei die Reaktion in Gegenwart eines natrisierenden Mittels in einem inerten Lösungsmittel durchgeführt wird,

— das so erhaltene Produkt danach durch Erhitzen in saurem Milieu zyklisiert wird,

— das so erhaltene Produkt der Formel I durch Einwirken einer Mineral- oder organischen Säure gegebenenfalls in eines seiner Salze übergeführt wird.

5. Vefahren zur Herstellung von Piperidindionderivaten der Formel I nach einem der Ansprüche 1 bis 4, dadurch gekennzeichnet, daß das Derivat der Formel I, worin $R_5$ für H steht, vor einer möglichen Salzbildung durch Alkylieren am Stickstoff nach bekannten Alkylierungsverfahren in eine Verbindung der Formel I, worin $R_5$ eine gerade oder verzweigte Alkylgruppe mit 1 bis 4 Kohlenstoffatomen darstellt, übergeführt wird.

6. Verfahren nach einem der Ansprüche 1 bis 5, dadurch gekennzeichnet, daß das so erhaltene Produkt in Form eines Diastereoisomeren, eines optischen Isomeren oder einer Mischung dieser Isomeren isoliert wird.

7. Verwendung der Piperidindionderivate der Formel I, erhalten nach einem der Ansprüche 1 bis 6, zur Herstellung von Medikamenten.

8. Verwendung von Piperidindionderivaten der Formel I, erhalten nach einem der Ansprüche 1 bis 6, zur Herstellung von Medikamenten zum Myokardschutz, welche Medikamente zwischen 50 und 800 mg der Derivate enthalten.